(19) Europäisches Patentamt / European Patent Office / Office européen des brevets

(11) **EP 4 559 458 A1**

(12) **EUROPEAN PATENT APPLICATION**
published in accordance with Art. 153(4) EPC

(43) Date of publication:
**28.05.2025 Bulletin 2025/22**

(21) Application number: **23843056.5**

(22) Date of filing: **21.07.2023**

(51) International Patent Classification (IPC):
*A61K 9/48* (2006.01)       *A61K 47/02* (2006.01)
*A61K 47/12* (2006.01)      *A61K 47/14* (2017.01)
*A61K 47/20* (2006.01)      *A61K 47/32* (2006.01)
*A61K 47/36* (2006.01)      *A61K 47/38* (2006.01)
*A61K 47/42* (2017.01)      *A61K 31/167* (2006.01)
*A61P 29/00* (2006.01)

(52) Cooperative Patent Classification (CPC):
**A61K 9/48; A61K 31/167; A61K 47/02;
A61K 47/12; A61K 47/14; A61K 47/20;
A61K 47/32; A61K 47/36; A61K 47/38;
A61K 47/42; A61P 29/00**

(86) International application number:
**PCT/JP2023/026717**

(87) International publication number:
**WO 2024/019133 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.07.2022 JP 2022117058
03.03.2023 JP 2023033057
31.05.2023 JP 2023090161**

(71) Applicant: **Qualicaps Co., Ltd.
Nara 639-1032 (JP)**

(72) Inventor: **ASO, Makoto
Yamatokoriyama-shi, Nara 639-1032 (JP)**

(74) Representative: **Kraus & Lederer PartGmbB
Thomas-Wimmer-Ring 15
80539 München (DE)**

(54) **MULTILAYERED ENTERIC RIGID CAPSULE**

(57)     A multilayered enteric hard capsule consisting of a first layer having no enteric properties and a second layer of a hard capsule having enteric properties is provided. The multilayered enteric hard capsule comprising a cap portion and a body portion, the cap portion and the body portion each consisting of a capsule shell comprising at least a first layer and a second layer in whole or in part, wherein the first layer contains a water-soluble base, the second layer contains an enteric polymer, and the second layer coats an outside of the first layer in whole or in part.

Fig. 2

(A)

(B)

(C)

**Description**

Technical Field

The present invention relates to a multilayered enteric hard capsule.

Background Art

The term "enteric" generally means a characteristic of a dosage form of formulations that are administered orally and hardly dissolve in the stomach. Also, the formulation has a characteristic of being easily dissolved after being transferred into the intestine. An enteric formulation does not release a drug active component in the stomach, which has a strong acidic environment, and releases a drug active component after the formulation is moved into the intestine. For this reason, an enteric formulation is mainly used for the purpose of protecting a drug active component from the gastric acid or gastric enzymes, or for the purpose of continuously releasing a drug active component utilizing the time during which the formulation is moved from the stomach to the small intestine.

In the field of pharmaceutical formulations, "enteric" is defined in almost the same way in Japan (Japanese Pharmacopoeia 18th Edition, 6.10 Dissolution Test, 4.3 Enteric Preparation), the United States (US Pharmacopeia Monograph <711> Dissolution 7, Delayed-Release Dosage Forms), and Europe (European Pharmacopeia, 2.9.3, Delayed-release dosage forms). In particular, Japan, Europe and the United States agree in that the formulations are required to have such a level of acid-resistance that they are not substantially dissolved for two hours at 37°C in an acidic environment (pH about 1.2, diluted hydrochloric acid solution). On the other hand, dissolution properties required in an intestine vary depending on whether a target site for release is a small intestine, colon, or large intestine, whether a drug release property is immediate-release or sustained-release, and so on.

When a dosage form is a tablet, an "enteric" formulation satisfying the above requirements is prepared by spraying and coating (spray coating) the tablet with a solution in which an enteric polymer is dissolved or dispersed.

When a dosage form is a hard capsule, an attempt has been made to make it enteric by coating. Such related arts include the following:

(1) a method in which the hard capsule filled with a content is spray-coated with an enteric polymer similar to that used for the tablet (Patent Literatures 1 and 2);
(2) a method in which an empty hard capsule is spray-coated with the enteric polymer and then filled with the content (Patent Literatures 3 to 5);
(3) a method of obtaining an enteric shell by dip-coating a dried hard capsule shell with an immersion solution containing the enteric polymer or an acid-resistant polymer again on the dried hard capsule shell in a process of producing the empty hard capsule (Patent Literature 6);
(4) a method of obtaining the enteric shell by dip-coating a commercially available hard capsule (Patent Literature 7);

and so on.

In addition, an attempt has been made to make a hard capsule shell itself enteric. Such related arts include the following:

(5) Using a conventional polymer such as gelatin, which is water-soluble and has high shell-forming ability, as a main component, and partially using the enteric polymer (Patent Literature 8);
(6) Neutralizing all or part of acid groups (mainly carboxyl groups) of the enteric polymer with a basic neutralizer in order to obtain a water-soluble derivative of the polymer (Patent Literatures 9 and 10);
(7) Using a gelling agent capable of imparting acid-resistance, such as gellan gum, instead of or together with the enteric polymer, thereby improving gelling property and shell performance while maintaining acid-resistance (Patent Literatures 11 and 12);

and so on.

Citation List

Patent Literature

[Patent Literature 1] US Patent No. 6309666

[Patent Literature 2] US Patent No. 7094425
[Patent Literature 3] WO2020/229178
[Patent Literature 4] WO2020/229192
[Patent Literature 5] Japanese Unexamined Patent Application No. 2003-325642
[Patent Literature 6] US Patent No. 3927195
[Patent Literature 7] Japanese Patent No. 5686802
[Patent Literature 8] Japanese Unexamined Patent Application No. 2006-16372
[Patent Literature 9] WO2019/245031
[Patent Literature 10] Japanese Translation of PCT International Application Publication No. 2015-518005
[Patent Literature 11] Japanese Unexamined Patent Application No. 2010-202550
[Patent Literature 12] Japanese Unexamined Patent Application No. 2009-196961

Summary of Invention

Technical Problem

However, the related arts have the following problems. In the related art described the above (1), the content is filled and a cap and body are fitted together before coating a surface, making a preparation process complex. There is a concern that when enteric coating is applied to the surface after filling the content into capsules, a coating solution is excessively sprayed on the surface of the filled capsules, causing the prepared filled capsules to adhere to each other, resulting in defective products and even forcing all filled capsules to be discarded. In addition, in order to form the enteric shell uniformly on the capsule surface, the spray coating must be performed for a long time, making the process less workable. In addition, since the spray coating method requires an application of hot air to dry the shell, it is difficult to apply the method to heat-sensitive contents. Furthermore, the burden of work due to the complexity of the preparation process is borne not by a manufacturer of the hard capsule, but by a manufacturer who fills the content. This would impair the convenience of the hard capsule as a form of formulation.

Next, in the related art described in the above (2), after preparing the enteric hard capsule formulation, it is necessary to separate the body from the cap to fill the content. In a step of separating the body from the cap, the spray-coated shell is torn, possibly resulting in a poor appearance due to non-uniformity in the cut area. In addition, there is a possibility that the original enteric properties may be impaired due to the non-uniformity of the site. Furthermore, in order to perform the spray coating efficiently without clogging, a concentration of solids in the coating solution is 5 to 10 wt%, and there is no description of a dip coating method.

Next, in the related art described in the above (3), it is described that the gelatin is used for an inner layer, and other materials such as calcium alginate, and modified cellulose derivatives, for example methyl cellulose and hydroxyalkyl-alkyl cellulose ethers, can be used for the inner layer. An outer layer is a layer that dissolves at pH 3.5 or higher and is formed of a material selected from "phenyl salicylate, butyl stearate, carnauba wax, shellac" or "methacrylic acid polymer, partial esters of maleic anhydride/alkene copolymers, HPMC phthalate, cellulose benzoacetate and cellulose acetate phthalate." As solvents used in examples of a dipping step for a second layer, an acetone/ethanol mixture, a methylene dichloride/methanol mixture, or an isopropanol/acetone mixture is used. It is illustrated that the capsule shell is formed by repeating a process of dipping and drying twice. Although the acid-resistance of the hard capsule shell is improved, there is concern that the acid-resistance of the outer layer is weak because the outer layer illustrated is thin, with a double wall thickness for the outer layer of from 20 to 60 μm, and an effect of the wall thickness is not shown.

Next, in the related art described in the above (4), an effect of a shell thickness is not shown, and only gelatin is shown as a shell of the inner layer.

Next, in the related art described in the above (5), since a water-soluble polymer is contained, there is a problem that acid-resistance is impaired compared to the hard capsule shell consisting of only an enteric polymer.

Next, in the related art described in the above (6), there is a concern about a decrease in water resistance. Since pharmaceutical preparations are generally taken with water, enteric formulations are also required to be water-resistant. Chlorinated enteric polymers or neutralized salts of enteric polymers dissolve in water, thus there is a problem that the content may be released in the stomach.

Next, in the related art described in the above (7), an acid-resistant base, unlike the enteric polymer, does not have strict pH-responsive properties, thus there are problems such as difficulty in controlling a release of the content at a targeted site.

Enteric polymers commonly distributed at present are used after being dispersed or dissolved in water (water based) or in a mixture of water and organic solvents (organic based). A water-based coating solution, in which nano- to micro-order enteric polymers are dispersed, can form the shell by being mixed with a plasticizer, but since it only forms a pseudo-shell, the shell is inadequate in terms of hardity or toughness as the shell for the hard capsule.

An object of the present invention is to provide a multilayered hard capsule consisting of a shell comprising a first layer (inner layer) having no enteric properties and a second layer (outer layer) having enteric properties.

Solution to Problem

The present invention includes the following embodiments.

Item 1.

A multilayered enteric hard capsule comprising a cap portion and a body portion, the cap portion and the body portion each comprising capsule shell comprising at least a first layer and a second layer in whole or in part, wherein the first layer contains a water-soluble base, the second layer contains an enteric polymer, and the second layer coats an outside of the first layer in whole or in part.

Item 2.

The multilayered enteric hard capsule according to item 1, wherein a thickness of the first layer of the capsule shell is 20 to 160 $\mu$m and a thickness of the second layer of the capsule shell is 20 to 70 $\mu$m.

Item 3.

The multilayered enteric hard capsule according to item 2, wherein the combined thickness of the first and second layers of the capsule shell is 70 to 150 $\mu$m.

Item 4.

The multilayered enteric hard capsule according to item 1, consisting of the second layer containing

(1) an enteric polymer whose content is 50 mass% or more when the total content of the shell components excluding moisture is set to 100 mass%, and
(2) a shell-forming aid.

Item 5.

The multilayered enteric hard capsule according to item 4, wherein the enteric polymer is one selected from the group consisting of hydroxypropyl methylcellulose acetate succinate, hydroxypropyl methylcellulose phthalate, carboxymethyl ethyl cellulose, and mixtures thereof.

Item 6.

The multilayered enteric hard capsule according to item 4, wherein percentage of acid residues neutralized in the enteric polymer in the capsule shell is 0.01% or less when the number of moles (number of groups) of acid residues in the enteric polymer before neutralization is set to 100%.

Item 7.

The multilayered enteric hard capsule according to item 4, wherein the shell-forming aid is at least one selected from the group consisting of a plasticizer, a surfactant, and a surface modifier.

Item 8.

The multilayered enteric hard capsule according to item 7, wherein the shell-forming aid is at least one selected from the group consisting of triethyl citrate, talc and sodium lauryl sulphate.

Item 9.

The multilayered enteric hard capsule according to item 1, wherein the water-soluble base is hydroxypropyl methylcellulose, gelatine, pullulan, or polyvinyl alcohol.

Item 10.

The multilayered enteric hard capsule according to item 1, wherein a dissolution rate of the content filled in the capsule is less than 1% after 2 hours in a dissolution test using a solution with pH 1.2.

Item 11.

The multilayered enteric hard capsule according to item 10, wherein the dissolution rate of the content filled in the capsule is less than 1% after 2 hours in the dissolution test using a solution with pH 5.0 or 6.0.

Item 12.

The multilayered enteric hard capsule according to item 10, wherein the dissolution rate of the content filled in the capsule is 80% or more after 30 minutes in the dissolution test using a solution with pH 6.8.

Item 13.

The multilayered enteric hard capsule according to item 1, wherein

the dissolution rate of the content filled in the capsule is less than 1% after 2 hours in the dissolution test using the solution with pH 1.2,
the dissolution rate of the content filled in the capsule is less than 1% after 2 hours in the dissolution test using the solution with pH 5.0 or 6.0, and
the dissolution rate of the content filled in the capsule is 80% or more after 30 minutes in the dissolution test using the solution with pH 6.8.

Item 14.

The multilayered enteric hard capsule according to item 1, wherein the content of the neutralizer per 100 g of capsule shell is 0.06 g or less.

Item 15.

An immersion solution for second layer for forming the second layer of the multilayered enteric hard capsule according to item 1, wherein an enteric polymer, or a solid component containing the enteric polymer and a shell-forming aid, is dissolved or dispersed in a mixed solvent of water and a hydrophilic organic solvent, the ratio of anhydrous ethanol in the mixed solvent being 50 mass% or more and 95 mass% or less, and a shear viscosity of the immersion solution for second layer being 150 cP or more at 25°C.

Item 16.

The immersion solution for second layer according to item 15, wherein a concentration of the enteric polymer in the immersion solution for second layer is 10 mass% or more.

Item 17.

The immersion solution for second layer according to item 15, wherein the content of the neutralizer in the immersion solution for second layer is 0.01 mass% or less when a total amount of the immersion solution for second layer is set to 100 mass%.

Item 18.

The immersion solution for second layer according to item 15, wherein the hydrophilic organic solvent is at least one selected from the group consisting of anhydrous ethanol, 2-propanol, and acetone.

Item 19.

A method of producing a multilayered enteric hard capsule including:

a step of preparing a hard capsule for a first layer of the multilayered enteric hard capsule; and

a step of immersing the hard capsule for the first layer in the immersion solution for second layer according to item 14, taking the hard capsule for the first layer out of the immersion solution for second layer, removing a mixed solvent in the immersion solution for second layer adhered to the hard capsule for the first layer, and molding the second layer of the hard capsule for the multilayered enteric hard capsule.

Item 20.

The method according to item 19, wherein the step of molding the second layer is a plurality of times.

Advantageous Effects of Invention

An advantage of the present invention is that it is possible to provide the multilayered hard capsule consisting of the capsule shell comprising the first layer containing at least one of the group consisting of HPMC, gelatine, polyvinyl alcohol and pullulan as an immediate-release base and the second layer containing the enteric polymer. An advantage of the present invention is that the second layer comprising the enteric polymer can be formed without using the gelling agent. An advantage of the present invention is that the multilayered enteric hard capsule can be filled with the content using a conventionally used capsule filling machine. According to the present invention, the dissolution rate in solutions of pH 4.0 to pH 6.0 is reduced.

Brief Description of Drawings

FIG. 1 shows an appearance of a multilayered enteric hard capsule.

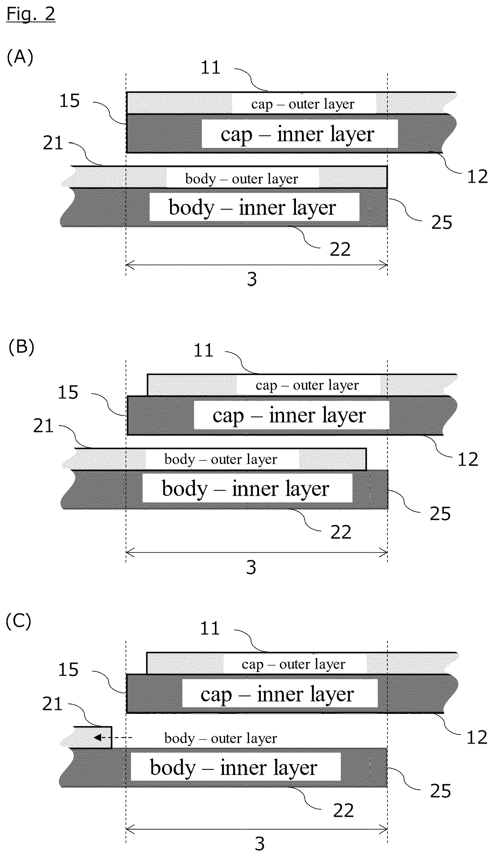

FIG. 2 is schematic diagrams of examples of structures of second layers 11 and 21 and first layers 12 and 22 constituting a capsule shell of the multilayered enteric hard capsule. FIG. 2A shows an aspect in which the second layer covers an entire surface of the first layer of a cap and body. FIG. 2B shows an aspect in which an area around a cut surface of the cap and body consists of only the first layer, while the second layer covers the entire surface of the first layer other than the cut surface. FIG. 2C shows an aspect in which the body of the portion covered with the cap consists of only the first layer.

FIG. 3 shows an example of the multilayered enteric hard capsule sealed with a band seal. FIG. 3A is an example of the structure shown in FIG. 2A to which the band seal is applied. FIG. 3B is an example of the structure shown in FIG. 2B to which the band seal is applied.

FIG. 4 shows a light microscopic image of a cross section of the multilayered enteric capsule to which the band seal is not applied.

FIG. 5 shows a light microscopic image of a cross section of the multilayered enteric capsule to which the band seal is applied.

FIG. 6-1 shows results of evaluations of solubility of each enteric polymer in water/anhydrous ethanol.

FIG. 6-2 shows results of evaluations of solubility of each enteric polymer in water/hydrophilic organic solvent.

FIG. 7-1 shows results of a dissolution test of the multilayered enteric hard capsule.

FIG. 7-2 shows results of a dissolution test of the multilayered enteric hard capsule.

FIG. 8-1 shows a composition of the multilayered enteric hard capsule in which the second layer consists of a plurality of layers, and results of the dissolution test.

FIG. 8-2 shows a composition of the multilayered enteric hard capsule in which the second layer consists of a plurality of layers, and results of the dissolution test.

FIG. 9 shows dissolution rates of the multilayered enteric hard capsule of the present disclosure in water, a solution of pH 5.0, or a solution of pH 6.0.

FIG. 10 shows dissolution rates of the multilayered enteric hard capsule comprising an outer layer prepared by dissolving enteric polymer by alkaline neutralization in water, the solution of pH 5.0, or the solution of pH 6.0.

FIG. 11 shows a concentration of ammonium ions in the hard capsule shell and the degree of neutralization of carboxyl groups of a base in the shell for comparative examples and examples.

Description of Embodiments

1. Multilayered Enteric Hard Capsule

In the present disclosure, a multilayered enteric hard capsule (sometimes simply referred to as a "hard capsule") has a capsule shell portion at least partially having a first layer and a second layer.

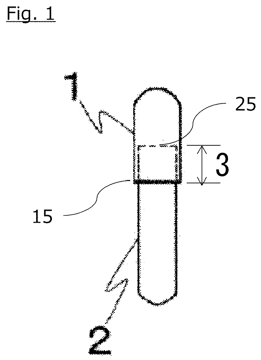

In the present disclosure, the term "hard capsule" refers to an empty capsule in which a content is filled into a produced capsule shell. Usually, the hard capsule is also called a two-piece capsule. These capsules are prepared by a so-called immersion method, in which a mold pin is immersed in a capsule preparation solution, pulled up, and the capsule preparation solution adhered to the mold pin is dried. In addition, as shown in FIG. 1, the hard capsule consists of a cap portion 1 and a body portion 2 consisting of a capsule shell, and the cap portion and the body portion are fitted together so that the content filled inside does not leak out of the capsule. An overlapped area formed by fitting the cap portion and the body portion is called a fitted portion 3 (indicated by a double arrow in FIG. 1). The "hard capsule" in the present invention has the same or similar shape as a commercially available conventional hard capsule which is intended to be orally administered to a human or animal subject.

The "hard capsule" of the present disclosure does not include a soft capsule produced by filling a content between two films and bonding the films to each other, a seamless capsule produced by dropping a content together with a shell solution into a solidification liquid, and a microcapsule prepared by incorporating therein an active ingredient by precipitation or emulsification of a base material.

In addition, in the present disclosure, an empty hard capsule is simply referred to as "hard capsule" or "capsule", and the hard capsule filled with the content is referred to as "hard capsule formulation".

In the present disclosure, in the "hard capsule", for example, as illustrated in FIG. 2, the capsule shell of the cap portion 1 comprises at least a second layer 11 and a first layer 12 in whole or in part. In addition, the shell of the body portion 2 also comprises at least a second layer 21 and a first layer 22 in whole or in part. Here, the "first layer" is intended to be a side that is in contact with a filling. In addition, the "second layer" covers the entire or part of an outer side of the first layer. Furthermore, the "second layer" may comprise a plurality of layers. The cap portion and the body portion may have the same or different layer structures. In other words, in the present disclosure, the hard capsule has a multilayered structure, preferably a two-layered structure, of the capsule shell in whole or in part. FIG. 2 shows an example of the structure of the capsule shell having the two-layered structure. The cap portion 1 and the body portion 2 are each prepared by the immersion method, as described below, and openings are cut after drying. A tip (end) of the cut portion of the capsule shell of the cap portion 1 is denoted by a code 15, and a tip (end) of the cut portion of the capsule shell of the body portion 2 is denoted by a code 25 (see FIG. 1).

FIG 2A is an aspect of the capsule shell consisting of the multilayered structure in which the whole of the first layers 12 and 22 is substantially covered with the second layers 11 and 21. The second layers 11 and 21 substantially cover the first layers 12 and 22 up to the end 15 of the cap portion 1 and the end 25 of the body portion 2 (alternatively, beyond the end 25). The term "substantially" indicates that portions of the first layers 12 and 22 of the ends 15 and 25 may not be unintentionally covered.

FIG. 2B is an aspect of the capsule shell consisting of the multilayered structure in which the second layers 11 and 21 do not reach the ends 15 and 25 of the first layers 12 and 22 in the cap portion 1 and the body portion 2. However, the second layers 11 and 21 at least reach the fitted portion 3 of the first layers 12 and 22. Such an aspect is useful because it allows the reuse of the capsule shell removed by cutting the opening after drying. Although FIG. 2B shows a state in which the first layers 12 and 22 of the cap portion 1 and the body portion 2 are covered with the second layers 11 and 21 to the same extent, a length of a non-covered portion of the first layer 12 in the cap portion 1 and a length of a non-covered portion of the first layer 22 in the body portion 2 may be different.

Figure 2C is an aspect of the capsule shell in which the multilayered structure in the cap portion 1 and the multilayered structure in the body portion 2 are different. As for the cap portion 1, the multilayered structure similar to the cap portion 1 shown in FIG. 2B is shown for illustrative purposes, but the capsule shell may also have the multilayered structure similar to the cap portion 1 shown in FIG 2B. On the other hand, in the body portion 2, the first layer 22 of the fitted portion 3 is substantially not covered with the second layer 21. Here, the term "substantially" indicates that the second layer 21 may be unintentionally adhered to the first layer 22 of the fitted portion 3. In this aspect, the first layer 22 may have a portion not covered with the second layer 21 in a predetermined extent distal from the end 15 of the cap portion 1 in addition to the fitted portion 3. The predetermined extent is, for example, from 50 $\mu$m to 3000 $\mu$m from the end 15.

Considering acid resistance, the capsule shell structure shown in FIG. 2B is preferable to the capsule shell structure shown in FIG. 2C, and the capsule shell structure shown in FIG. 2A is even more preferable.

FIG. 2 shows an example in which the cap portion and the body portion have the same structure. However, for example, the cap portion shown in FIG. 2A may be combined with the body portion shown in FIG. 2B or FIG. 2C. Also, the cap portion shown in FIG. 2B may be combined with the body portion shown in FIG. 2A or FIG. 2C.

In the present disclosure, the term "enteric hard capsule" refers to the hard capsule in which the shell of a capsule main body itself has "enteric" properties that satisfy the following conditions.

In other words, "enteric" refers to the properties that satisfy at least the following condition (i).

(i) In a dissolution test described in the Japanese Pharmacopoeia, 18th Edition (hereinafter sometimes simply referred to as the "18th Pharmacopoeia"), a dissolution rate of a content when a test subject is immersed in a first fluid at 37°C ± 0.5°C for 2 hours is 10% or less, preferably 5% or less, more preferably 2% or less, even more preferably less than 1%. A pH of the first fluid is about 1.2. The first fluid may be prepared, for example, by adding 7.0 ml of hydrochloric acid and water to 2.0 g of sodium chloride to adjust its volume to 1,000 ml.

In addition, a dissolution pH of the enteric polymer is strictly controlled, and it is preferable that the enteric polymer does not dissolve below the dissolution pH. For example, HPMCAS-L, which is expected to dissolve in a small intestine, dissolves at pH 5.5 or higher, but not at pH 5.0 or lower. Therefore, in the dissolution test described above, the dissolution rate of the content when the test subject is immersed in a solution of pH 5.0 at 37°C ± 0.5°C for 2 hours is 10% or less, preferably 5% or less, more preferably 2% or less, even more preferably less than 1%. The solution of pH 5.0 may be prepared using, for example, 0.05 mol/L of disodium hydrogen phosphate and 0.025 mol/L of citric acid.

In addition, HPMCAS-H, which is expected to dissolve in a large intestine, dissolves at pH 6.5 or higher, but not at pH 6.0 or lower. Therefore, in the dissolution test described above, the dissolution rate of the content when the test subject is immersed in a solution of pH 6.0 at 37°C ± 0.5°C for 2 hours is 10% or less, preferably 5% or less, more preferably 2% or less, even more preferably less than 1%. The solution of pH 6.0 may be prepared using, for example, 0.05 mol/L disodium hydrogen phosphate and 0.025 mol/L citric acid.

Furthermore, since pharmaceuticals are generally taken with water, it is preferable that the enteric polymer also have water resistance in order to dissolve at any site. Therefore, in the dissolution test described above, the dissolution rate of the content when the test subject is immersed in purified water at 37°C ± 0.5°C for 2 hours is 10% or less, preferably 5% or less, more preferably 2% or less, even more preferably less than 1%.

The term "enteric" preferably satisfies the above condition (i) as well as the following condition (ii). (ii) In the dissolution test described above, the content is released when the test subject is immersed in a second fluid at 37°C ± 0.5°C. A pH of the second fluid is about 6.8. The second fluid may be prepared, for example, by adding 118 ml of 0.2 mol/l sodium hydroxide solution and water to 250 ml of 0.2 mol/l potassium dihydrogen phosphate solution to adjust its volume to 1000 ml. Here, the time for measuring the dissolution rate of the content in the second fluid is not limited. For example, if it is required that the content is released relatively quickly after reaching an intestine, the dissolution rate after 30 minutes of immersion of the test subject in the second fluid is 50% or more, preferably 70% or more, and even more preferably 80% or more.

The dissolution test may be performed in accordance with a dissolution test method specified in the 18th Pharmacopoeia (17th Pharmacopoeia, 6.10-1.2 Paddle Method (paddle rotation speed: 50 rpm), with a sinker corresponding to each size.

The content used in the dissolution test is not limited as long as the content itself is rapidly dissolved in a test solution and can be quantified by known methods. An example is acetaminophen.

Sizes of the multilayered enteric hard capsule include No. 000, No. 00, No. 0, No. 1, No. 2, No. 3, No. 4, No. 5, No. 9, and the like, as with the hard capsule currently commercially available, but any size of the multilayered enteric hard capsule may be produced in the present invention.

(1) First Layers 12 and 22

The first layers 12 and 22 of the multilayered enteric hard capsule consist of the shell used in a common hard capsule. The shell of the common hard capsule is a capsule shell that dissolves in a stomach and the content is released. In other words, in the dissolution test described in the Japanese Pharmacopoeia, 18th Edition (hereinafter sometimes simply referred to as the "18th Pharmacopoeia"), the dissolution rate of the content when the test subject is immersed in the first fluid at 37°C ± 0.5°C for 30 minutes is 80% or more.

The shell constituting the first layers 12 and 22 contains a water-soluble base as a base. The water-soluble base is a polymer and includes, for example, water-soluble cellulose compounds, gelatin, pullulan, polyvinyl alcohol, polyvinyl alcohol copolymers, and mixtures thereof. The water-soluble base is preferably hydroxypropyl methylcellulose or gelatin.

The hydroxypropyl methylcellulose to be used in the present invention includes hypromellose of substitution grades (substitution types) 2910, 2906 and 2208 specified in the 18th Pharmacopoeia.

In addition, the hydroxypropyl methylcellulose according to the present invention includes the hypromellose that is approved for use as a food additive in Japan.

Commercially available hydroxypropyl methylcellulose may include, Japanese Pharmacopoeia METOLOSE® series and METOLOSE® series for food additives available from Shin-Etsu Chemical Co., Ltd., AnyCoat-C® or AnyAddy® series available from Lotte Fine Chemical Co., Ltd., METHOCEL™ series available from IFF, Benecel™ series available from Ashland Inc, and the like.

When the water-soluble base is the hydroxypropyl methylcellulose, a gelling agent or the gelling agent and a gelling aid can be used to form the shell constituting the first layers 12 and 22.

Examples of the gelling agent include those that can make the hard capsule preparation solution gel in combination with the gelling aid such as carrageenan and gellan gum. These can be used singly, or in combination of two.

Among the above gelling agents, carrageenan, which has a high gel strength and can provide an excellent gelation effect in the presence of specific ions even when used in a small amount, is the optimum gelling agent. In general, three types of carrageenan are known: kappa-carrageenan, iota-carrageenan and lambda-carrageenan. In the present invention, the kappa- and iota-carrageenans having relatively high hardity and gelling ability can be suitably used, more suitably the kappa-carrageenan can be used. The gellan gum also can be classified into acylated gellan gum (native gellan gum) and deacylated gellan gum depending on the presence or absence of acylation, but both can be used in the present invention without distinction.

Commercially available carrageenan may include GENUGEL® series, GENUVISCO® series, GENULACTA® series, GENU® series, and GENUTINE® series available from CP Kelco, SOAGEENA™ and SOAACE™ available from Mitsubishi Chemical Corporation, Gelcarin® series, SeaSpen® series, and Viscarin® series available from IFF, and the like.

Commercially available gellan gum may include KELCOGEL® series available from CP Kelco, NEWGELIN® series available from Mitsubishi Corporation Life Sciences Limited, and the like.

A content of the gelling agent in the shell constituting the first layers 12 and 22 is not limited as long as the shell can be molded by a cooling gel method. The content of the gelling agent may be 0.05 to 10 mass%, preferably 0.1 to 5.0 mass%, more preferably 0.2 to 2.5 mass%, when the total component of the shell constituting the first layers 12 and 22, excluding moisture, is set to 100 mass%.

The gelling aid can be selected according to a type of the gelling agent to be used. The gelling aid has an effect of accelerating the gelling of the gelling agent. Alternatively, it may contribute to the acceleration of gelation by directly acting on a cellulose compound to raise or lower its gelation temperature or cloud point temperature. When carrageenan is used as a gelling agent, the following gelling aids can be used in combination with the gelling agent. For kappa-carrageenan, examples include compounds capable of producing one or more of sodium ions, potassium ions, ammonium ions and calcium ions in an aqueous solution, such as sodium chloride, potassium chloride, potassium phosphate, ammonium chloride, ammonium acetate and calcium chloride. Preferably, it is a compound that produces the sodium ions, potassium ions or calcium ions in the aqueous solution. For iota-carrageenan, examples include compounds capable of donating calcium ions in water, such as calcium chloride. When gellan gum is used as a gelling agent, examples of gelling aids that can be used in combination with the gelling agent include compounds capable of donating one type or two or more types of ions selected from sodium ions, potassium ions, calcium ions and magnesium ions in water, such as sodium chloride, potassium chloride, calcium chloride and magnesium sulfate. In addition, an organic acid or a water-soluble salt thereof, such as citric acid or sodium citrate, can also be used.

The content of the gelling aid in the shell constituting the first layers 12 and 22 may be set according to the content of the gelling agent. The content of the gelling aid may be 0.05 to 10 mass%, preferably 0.1 to 5.0 mass%, more preferably 0.2 to 2.5 mass%, when the total component of the shell constituting the first layers 12 and 22, excluding moisture, is set to 100 mass%.

In the present invention, carrageenan, especially kappa-carrageenan, is a suitable gelling agent to be used in combination, and potassium chloride is a suitable gelling aid to be used in combination with it.

The content of the hydroxypropyl methylcellulose in the shell constituting the first layers 12 and 22 is the remainder, excluding the content of the gelling agent; the gelling agent and gelling aid; the plasticizer, lubricant, colorant, and light shielding agent described below, when the total component of the shell constituting the first layers 12 and 22, excluding moisture, is set to 100 mass%.

The hard capsule using the hydroxypropyl methylcellulose as the base is well known and available on a commercial basis. Examples of the hard capsule using the hydroxypropyl methylcellulose as the base may include Quali-V® (Qualicaps Co.,Ltd.), Vcaps® series (Lonza K.K.), Embocaps VG® series (Suheung), ACGCAPS™ series (ACG), and the like.

The gelatin used in the present disclosure is an animal-derived gelatin, and the hard capsule using gelatin as the base is well known and is also available on a commercial basis. Examples of the hard capsule using the gelatine as the base may include Quali-G® (Qualicaps Co.,Ltd.), Coni-Snaps® (Lonza K.K.), Embocaps® series (Suheung), ACGCAPS™ series (ACG), and the like.

The hard capsule containing the pullulan, polyvinyl alcohol, or polyvinyl alcohol copolymer as the base is well known. Examples of the hard capsule also available on a commercial basis may include Plantcaps™ (Capsugel Japan, Inc.), which is a pullulan capsule, and PONDAC™ (Daido Chemical Industry Co., Ltd.), which is a polyvinyl alcohol copolymer capsule.

The shell constituting the first layers 12 and 22 may contain the plasticizer, lubricant, colorant, light shielding agent, and residual moisture, in addition to the water-soluble base, gelling agent, gelling aid, and the like.

When the shell constituting the first layers 12 and 22 contains the plasticizer, lubricant, colorant, light shielding agents, and the like, it is 10 mass% or less, preferably 8 mass% or less, and more preferably 6 mass% or less when the total mass of the components other than the residual solvent components in the shell constituting the first layers 12 and 22 is set to 100 mass%.

The plasticizer is not particularly limited as long as it can be used in pharmaceutical or food compositions, however, suitable substances are those that generally have a molecular weight (Mw) of 100 to 20,000 and have one or a plurality of hydrophilic groups, such as a hydroxyl, ester, or amino group, in one molecule. Examples thereof may include dioctyl adipate, polyester adipate, epoxidized soybean oil, epoxyhexahydrophthalic acid diester, kaolin, triethyl citrate, glycerin, glycerin fatty acid ester, sesame oil, dimethyl polysiloxane-silicon dioxide mixture, D-sorbitol, medium-chain fatty acid triglyceride, corn starch-derived sugar alcohol liquid, triacetin, concentrated glycerin, castor oil, phytosterol, diethyl phthalate, dioctyl phthalate, dibutyl phthalate, butyl phthalyl butyl glycolate, propylene glycol, polyoxyethylene (105) polyoxypropylene (5) glycol, polysorbate 80, macrogol 1500, macrogol 400, macrogol 4000, macrogol 600, macrogol 6000, isopropyl myristate, dioctyl adipate-soybean oil mixture, glycerin monostearate, and isopropyl linoleate.

The lubricant is not particularly limited as long as it can be used in pharmaceutical products or food compositions. Examples include calcium stearate, magnesium stearate, sodium stearyl fumarate, carnauba wax, starch, sucrose fatty acid esters, light anhydrous silicic acid, macrogol, talc, and hydrogenated vegetable oils.

The colorant and light-shielding agent are not particularly limited as long as they can be used in pharmaceutical products or food compositions. Examples may include powdered gambir tannin, turmeric extract, methylrosaniline chloride, yellow iron oxide, yellow iron sesquioxide, Opaspray K-1-24904, orange essence, brown iron oxide, carbon black, caramel, carmine, carotene liquid, β-carotene, photosensitizer 201, licorice extract, gold leaf, Sasa veitchii extract, black iron oxide, light anhydrous silicic acid, Daemonorops draco (kekketsu), zinc oxide, titanium oxide, calcium carbonate, iron sesquioxide, disazo yellow, Food Blue No. 1 and its aluminum lake, Food Blue No. 2 and its aluminum lake, Food Yellow No. 4 and its aluminum lake, Food Yellow No. 5 and its aluminum lake, Food Green No. 3 and its aluminum lake, Food Red No. 2 and its aluminum lake, Food Red No. 3 and its aluminum lake, Food Red No. 102 and its aluminum lake, Food Red No. 104 and its aluminum lake, Food Red No. 105 and its aluminum lake, Food Red No. 106 and its aluminum lake, Food Red No. 40 and its aluminum lake, talc, sodium copper chlorophyllin, copper chlorophyll, powdered hull-less barley green tea extract, hull-less barley green tea extract, phenol red, sodium fluorescein, d-borneol, malachite green, octyldodecyl myristate, methylene blue, medicinal carbon, riboflavin butyrate, riboflavin, powdered green tea, manganese ammonium phosphate, riboflavin sodium phosphate, rose oil, turmeric oleoresin, chlorophyll, carminic acid, water-soluble annatto, and the like.

(2) Second Layers 11 and 21

The second layers 11 and 21 impart an enteric property to the hard capsule of the present disclosure as described above.

The enteric property can be imparted by dipping the hard capsule comprising the first layers 12 and 22 in the above section 2.1 (1) in an immersion solution for second layer containing the enteric polymer by the immersion method, pulling up, and drying to form a shell constituting the second layers 11 and 21.

The enteric polymer used in the present invention is an enteric cellulose compound (polymer). Specifically, it refers to a compound in which a hydrogen atom of a hydroxy group of cellulose is etherified with phthalic acid, acetic acid, succinic acid or the like containing a carboxyl group. Examples of the enteric cellulose compound may include hydroxypropyl methylcellulose acetate succinate (HPMCAS), hydroxypropyl methylcellulose phthalate (HPMCP), and carboxymethyl cellulose (CMEC).

The HPMCAS, also called hypromellose acetate succinate, is obtained by reacting HPMC (hypromellose) with acetic anhydride and succinic anhydride, and introducing an acetyl group ($-COCH_3$) and a succinoyl (also called "succinyl") group ($-COC_2H_4COOH$).

Examples of HPMCAS products are available, for example, as AQOAT® series products from Shin-Etsu Chemical Co. This series includes three substitution grades as AS-L, AS-M, and AS-H, depending on the degree of substitution of the succinoyl and acetyl groups. In the order of grade (L, M or H), the content of the sucinoyl group is controlled to be lower while the content of the acetyl group is controlled to be higher, and the dissolution pH is set to be higher. The dissolution pH of the AS-L, M, and H are approximately pH 5.5, pH 6.0, and pH 6.5, respectively. The AS-L, M, and H are available as coarse AS-LG, MG, and HG, and AS-LF, MF, and HF with an average particle size of 10 μm or less. When dissolved in hydrophilic organic solvent, the AS-LG, MG, and HG are preferred. Products with various degrees of substitution are available from Ashland as one of AquaSolve® series.

The HPMCP, also called hypromellose phthalate, is obtained by reacting the HPMC (hypromellose) with phthalic anhydride to make it enteric, and its dissolution pH may vary depending on an amount of carboxybenzoyl groups attached. The carboxybenzoyl group itself is hydrophobic and acid-resistant, whereas the carboxybenzoyl group dissociates in a weakly acidic to neutral range to allow the HPMCP to dissolve.

As examples of products, two varieties of HP-55 (substitution type 200731) and HP-50 (substitution type 220824), which have different dissolution pHs, and HP-55S, which has a higher degree of polymerization than the HP-55 and superior film strength, are available from Shin-Etsu Chemical Co., Ltd. and Lotte Fine Chemical Co., Ltd. The dissolution pH of the HP-50 and HP-55 are approximately pH 5.0 and pH 5.5, respectively.

The CMEC, also called carboxymethylethylcellulose, is an ether obtained by partial carboxymethylation and ethylation of hydroxyl groups of cellulose. It is used as an enteric polymer because it is difficult to dissolve at low pH and soluble at high pH.

As examples of products, CMEC® is available from FREUND Corporation.

Said enteric polymer is preferably one selected from the group consisting of the HPMCAS, HPMCP, CMEC and mixtures thereof, and is more preferably the HPMCAS.

Said enteric polymer is not substantially neutralized in the capsule shell and in the immersion solution for second layer. Acid residues such as carboxyl groups in the enteric polymer are neutralized with a compound that shows alkalinity in water. For example, when the carboxyl groups are neutralized with NaOH, KOH, or $NH_3$, they can exist stably as chlorinated groups such as -COONa, -COOK, and $-COONH_4$ in the capsule shell or in the immersion solution for second layer. The "not substantially neutralized" means that the percentage of these neutralized acid residues is, for example, 0.01% or less, 0.001% or less, 0.0001% or less, 0.00001% or less, or 0.000001% or less, when the number of moles (number of groups) of the acid residues in the enteric polymer before neutralization is set to 100%.

In order to neutralize the acid residues, the content of a neutralizer per 100 g of the shell containing the first layers 12 and 22 and the second layers 11 and 21 is 0.06 g or less, preferably 0.01 g or less, more preferably 0.001 g or less, and even more preferably 0.001 g or less.

The shell constituting the second layers 11 and 21 may be added with a shell-forming aid in addition to the enteric polymer described above. The triethyl citrate (TEC), the talc, and sodium lauryl sulfate (SLS) and mixtures thereof may be used as said shell-forming aid. When the total component of the shell constituting the second layers 11 and 21, excluding moisture, is set to 100 mass%, the content of each component is, for example: when the TEC is selected, specifically 5 mass% or more and 20 mass% or less; when the talc is selected, specifically 5 mass% or more and 40 mass% or less; when the sodium lauryl sulfate is selected, 1 mass% or more and 3 mass% or less.

The shell constituting the second layers 11 and 21 may contain the plasticizer, lubricant, colorant, light shielding agent, residual moisture, and the like. The plasticizer, lubricant, colorant, and light shielding agent illustrated in the above section 1.1 (1) can be used in the illustrated contents.

The content of the enteric polymer in the shell constituting the second layers 11 and 21 is the remainder, excluding the content of the gelling agent; the gelling agent and gelling aid; the plasticizer, lubricant, colorant, and light shielding agent, when the total component of the shell constituting the second layers 11 and 21, excluding moisture, is set to 100 mass%.

In both the first layers 12 and 22 and the second layers 11 and 21, the residual solvent after preparation of the capsule shell is preferably 0.5 mass% or less when the mass of the capsule shell is set to 100 mass%.

A thickness of the shell (including the first layer and second layer) of the multilayered enteric hard capsule is typically 50 to 250 $\mu$m, preferably 70 to 150 $\mu$m, and more preferably 80 to 120 $\mu$m, as with the hard capsule currently commercially available.

A thickness of the first layers 12 and 22 is from 20 to 160 $\mu$m, more preferably from 40 to 130 $\mu$m, and even more preferably from 60 to 120 $\mu$m.

A thickness of the shell constituting the second layers 11 and 21 is 20 to 100 $\mu$m, preferably 30 to 75 $\mu$m, and more preferably 30 to 70 $\mu$m.

It is preferred that, as the shell of the multilayered enteric hard capsule, a thickness of the first layer is 20 to 50 $\mu$m and a thickness of the second layer is 50 to 100 $\mu$m, and a combined thickness of the first and second layers is 70 to 150 $\mu$m. Alternatively, it is preferred that, as the shell of the multilayered enteric hard capsule, the thickness of the first layer is 50 to 100 $\mu$m and the thickness of the second layer is 20 to 50 $\mu$m, and the combined thickness of the first and second layers is 70 to 150 $\mu$m.

2. Immersion Solution for Preparing Hard Capsule Shell

(1) Immersion Solution for First Layer

Since the first layers 12 and 22 are known hard capsule sells, an immersion solution for forming the first layers 12 and 22 (hereinafter referred to as "immersion solution for first layer") is known.

The immersion solution for first layer can be prepared, for example, by dissolving a substance selected from the HPMC or gelatin in purified water heated to about 70 to 80°C, after dissolving the gelling agent and gelling aid and components other than the water-soluble base as needed, and then cooling this mixture to a desired temperature of the immersion solution (typically 35 to 65°C, more preferably 40 to 60°C).

(2) Immersion Solution for Second Layer

An immersion solution for forming the second layers 11 and 21 (hereinafter referred to as "immersion solution for second layer") can be prepared by the following method.

Since said enteric polymer is insoluble in the purified water, a solvent containing the hydrophilic organic solvent is used to obtain a uniform solution. Purified water is intended to be "purified" water by a single or combined system of ion exchange, distillation, reverse osmosis or ultrafiltration.

The hydrophilic organic solvent is not particularly limited as long as it can be used in pharmaceutical products or food compositions, and examples of it may include ethanol, acetone, 1-propanol, 2-propanol, diethyl ether, dichloromethane, and methanol. The hydrophobic organic solvent is preferably ethanol, and the ethanol is preferably anhydrous ethanol.

The hydrophilic organic solvent described above preferably has a low risk to human health. For example, ethanol, acetone, and 2-propanol, which are solvents that should be regulated by GMP or other quality standards and fall into Class 3 (18th Pharmacopoeia, 2.46 Residual Solvents), are preferred.

The immersion solution for second layer can be prepared by adding the enteric polymer or a solid component containing the enteric polymer and shell-forming aid to a mixed solvent of water and the hydrophilic organic solvent at room temperature, then stirring for about several hours to dissolve or disperse the enteric polymer or the solid component containing the enteric polymer and shell-forming aid. A content of the enteric polymer in the immersion solution for second layer is 10 mass% or more and 30 mass% or less, preferably 11 mass% or more and 25 mass% or less, more preferably 13 mass% or more and 20 mass% or less, when the immersion solution for second layer is set to 100 mass%. A solvent of the immersion solution for second layer is preferably a mixed solvent of water and anhydrous ethanol, and the ethanol ratio in said mixed solvent (ethanol content is the ethanol content converted to anhydrous ethanol) is specifically 50 mass% or more and 95 mass% or less, preferably 60 mass% or more and 93 mass% or less, more preferably 70 mass% or more and 90 mass% or less.

The immersion solution for second layer is substantially free of a neutralizer such as an alkaline substance. Examples of the neutralizer may include potassium hydroxide, sodium hydroxide, calcium hydroxide, ammonium hydroxide, ammonia, and the like. Substantially free of the neutralizer such as the alkaline substance means that the neutralizer is 0.01 mass% or less, 0.001 mass% or less, or 0.0001 mass% or less when the total of the immersion solution for second layer is set to 100 mass%.

The shear viscosity of the immersion solution for second layer at 25°C and a shear rate of 1 sec$^{-1}$ is specifically 150 cP or more and 10000 cP or less, preferably 300 cP or more and 5000 cP or less.

3. Method of Preparing Multilayered Enteric Hard Capsule

The present disclosure relates to a molding method for producing the multilayered enteric hard capsule.

(1) Molding of First Layer

Molding of the first layers 12 and 22 of the multilayered enteric hard capsule can be performed by the immersion method in the same way as the preparation of the hard capsule using a general water-soluble base. A mold pin (capsule molding pin), which serves as a mold for the capsule, is immersed in the immersion solution for first layer described in 2.1 (1) above, and then pulled up. When pulled up, the temperature of the immersion solution for first layer, which adheres to the pin, drops and it thickens. By drying it, the desired capsule shape and thickness is obtained. The shell of the general hard capsule is cut into predetermined lengths after drying and pulling out the mold pin, while in the multilayered enteric hard capsule described above, this cutting is done after the second layer is formed in the following step.

However, the first layer of the multilayered enteric hard capsule may be a hard capsule containing a water-soluble base, which is available on a commercial basis. In addition, the molding of the first layer and the molding of the second layer described below need not be performed by the same facility. Therefore, the term "preparing the hard capsule for the first layer" as used herein includes molding the hard capsule for the first layer, purchasing the hard capsule for the first layer and acquiring the hard capsule for the first layer from another facility.

(2) Molding of Second Layer

The second layers 11 and 21 of the multilayered enteric hard capsule are also molded by the immersion method. The hard capsule for the first layer prepared in the above section 3.1 (1) is immersed in the immersion solution for second layer described in section 2.2 (2) above, and the entire outside or part of the outside of the hard capsule for the first layer is taken out of the immersion solution for second layer. This step allows the immersion solution for second layer to adhere to the hard capsule for the first layer. Since the thickness required for the second layer is smaller than that of the first layer, an amount of the immersion solution for second layer can be less adhered to the second layer, and further, the hydrophilic organic solvent as the solvent quickly dries and rapidly thickens to obtain a desired capsule shape and thickness. The mixed solvent in the immersion solution for second layer adhered to the hard capsule for the first layer is removed, and the second layer of the hard capsule of the multilayered enteric hard capsule is molded. The removal of the mixed solvent is preferably by drying, more preferably by drying at room temperature (about 18°C. to 30°C.). After molding the second layer, the immersion process is repeated a plurality of times using the immersion solution for second layer having the same composition or the immersion solution for second layer having a different composition, so that the second layer can have the multilayered structure and the thickness of the second layer can be adjusted to the desired thickness.

The shell of the dried multilayered enteric hard capsule is pulled from the mold pin after drying and cut into predetermined lengths.

4. Filling of Content into Multilayered Enteric Hard Capsule

A content may be filled into the multilayered enteric hard capsule using a known capsule filling machine, for example, a fully-automatic capsule filling machine (model name: LIQFIL super 80/150, manufactured by Qualicaps Co., Ltd.), a capsule filling and sealing machine (model name: LIQFIL super FS, manufactured by Qualicaps Co., Ltd.) or the like. The body portion and the cap portion of the hard capsule thus obtained are joined to each other by covering the body portion with the cap portion to fit the body portion and the cap portion with each other after the content is filled into the body portion to obtain the hard capsule formulation.

5. Sealing of Hard Capsule Formulation after Filling

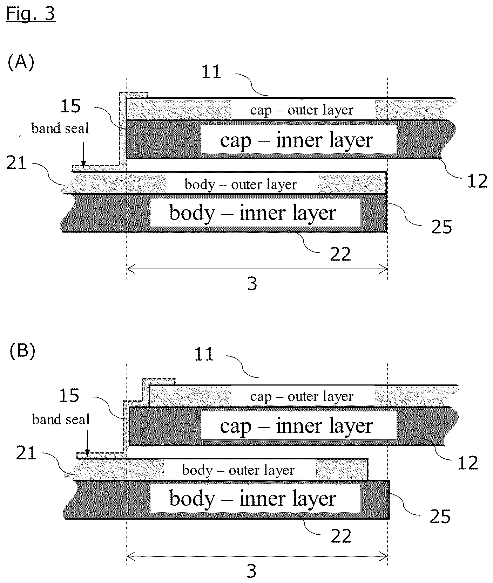

If necessary, the hard capsule formulation may be made tamper-proof by using appropriate techniques to seal at least the end 15 of the cap portion 1 and the body portion 2. Typically, sealing or banding techniques are used, as shown in FIG. 3. FIG. 3A is an example of the structure shown in FIG. 2A to which a band seal is applied. FIG. 3B is an example of the structure shown in FIG. 2B to which the band seal is applied. These techniques are well known to a person skilled in the art of capsule. An enteric hard capsule formulation can be obtained by sealing the fitted portion by applying a sealing agent of polymer solution to the surface of the body portion and the surface of the cap portion once or a plurality of times, preferably once or twice, in the circumferential direction of the body portion and the cap portion at a constant width centered on the end 15 of the cap portion 1. As the polymer solution, a solution obtained by dispersing the enteric polymer in water or by dissolving the enteric polymer in the mixed solvent of water and the hydrophilic organic solvent may be used.

During capsule sealing, the preparing solution for forming the band seal may be used generally at room temperature or under heating. From the viewpoint of preventing liquid leakage of the hard capsule, it is desired to use the preparing solution for forming the band seal within a temperature range of preferably from about 23 to 45°C, more preferably from about 23 to 35°C, most preferably from about 25 to 35°C. The temperature of the preparing solution for forming the band seal may be adjusted by a method known per se, such as a panel heater or a hot-water heater. However, it is preferable to adjust the temperature by, for example, a circulating hot-water heater or a seal pan unit of the above-described integrated capsule filling and sealing machine which is remodeled into a circulating hot-water heater type, since the temperature range can be adjusted delicately.

The enteric hard capsule formulation of the present invention thus obtained is designed so that when administered and ingested into a human or animal body, it is acid-resistant in a stomach and migrates mainly into an intestine, where the capsule shell dissolves and the content is released. This makes it suitable as a formulation filled with pharmaceuticals and foods that are not preferably released in the stomach.

Examples

Hereinafter, the present invention will be described in more detail with reference to examples. However, the present invention should not be construed as limited to the examples.

I. Materials to Be Used

Materials to be used in examples, comparative examples and formulation examples are as described below.

1. Gelatine

As a gelatine, 230NV available from Nitta Gelatin Inc. was used (Hereinafter referred to as "gelatin").

2. Nonionic Water-Soluble Cellulose Compound

As a hydroxypropyl methylcellulose (HPMC), a combination of TC-5M and TC-5R series, or HPMC with substitution grade 2208 (viscosity grade 4.0) available from Shin-Etsu Chemical Co., Ltd. was used (hereinafter referred to as "TC-5M" and "TC-5R" respectively).

3. Pullulan

Pullulan available from Tokyo Chemical Industry Co., Ltd. was used.

4. Polyvinyl Alcohol

As a polyvinyl alcohol, EG-05P available from Mitsubishi Chemical Corporation was used (hereinafter sometimes referred to as "EG-05P" or "PVA").

5. Gelling Agent

As a gelling agent, GENUGEL SWG-J CP available from CP Kelco as a κ-carrageenan was used (hereinafter referred to as "CA"). As a gelling aid, potassium chloride was used (hereinafter referred to as "KCl"). As a gellan gum, KELCOGEL® available from CP Kelco was used (hereinafter referred to as "GE").

6. Enteric Polymer

(1) Hydroxypropyl Methylcellulose Acetate Succinate (HPMCAS)

LG, MG, and HG grades of AQOAT series available from Shin-Etsu Chemical Co., Ltd. were used (hereinafter referred to as "AS-LG", "AS-MG" and "AS-HG" respectively).

(2) Hydroxypropyl Methylcellulose Phthalate (HPMCP)

HP-50, HP-55, and HP-55S grades of HPMCP series available from Shin-Etsu Chemical Co., Ltd. were used (hereinafter referred to as "HP-50", "HP-55" and "UP-55S" respectively).

(3) Carboxymethyl Ethyl Cellulose (CMEC)

Carboxymethyl cellulose available from FREUND Corporation was used (hereinafter referred to as "CMEC").

7. Solvent

As an ethanol, anhydrous ethanol "NIKKO" available from Nikko Pharmaceutical Co., Ltd. was used (hereinafter referred to as "EtOH"). As an acetone, acetone for medicine test available from FUJIFILM Wako Pure Chemical Corporation was used. As a 2-propanol, 2-propanol for medicine test available from FUJIFILM Wako Pure Chemical Corporation was used (hereinafter referred to as "IPA"). As an ammonia solution, ammonia solution for medicine test available from FUJIFILM Wako Pure Chemical Corporation was used.

8. Others

As a titanium oxide, TIPAQUE A-100 available from ISHIHARA SANGYO KAISHA, LTD. was used (hereinafter referred to as "$TiO_2$"). As a talc, MICRO ACE P-3 available from NIPPON TALC CO., LTD. was used (hereinafter referred to as "Talc"). As a sodium lauryl sulphate, sodium lauryl sulphate [for production only] available from KOKUSAN CHEMICAL Co.,Ltd. was used (hereinafter referred to as "SLS"). As a Blue No. 1, Blue No. 1 available from Daiwa Dyestuff Mfg. Co., Ltd. was used (hereinafter referred to as "Blue No. 1"). As a Red No. 102, Red No. 102 available from Daiwa Dyestuff Mfg. Co., Ltd. was used (hereinafter referred to as "Red No. 102").

9. Reagents for Dissolution test

As an acetaminophen, acetaminophen available from YAMAMOTO CORPORATION was used (hereinafter referred to as "AA"). As a lactose, Pharmatose 100M available from DFE Pharma was used, and as a sodium starch glycolate, EXPLOTAB available from JRS Pharma was used.

II. Method of Preparing Immersion Solution

A method of preparing an immersion solution to be used in the examples, comparative examples and formulation examples are as described below.

1. Gelatine Immersion Solution

183.33 g of gelatine was added to 316.67 g of purified water and allowed to stand for about 1 hour to swell. The swollen solution was allowed to stand at 60°C and confirmed to be completely dissolved. The solution was further allowed to stand at 60°C to confirm that the bubbles had completely disappeared. 31.8 g of 22 mass% $TiO_2$ dispersion at 55°C and 100.3 g of purified water at 55°C were added, and the solution was stirred for about 15 minutes to obtain an aqueous gelatine solution.

2. HPMC Immersion Solution

An HPMC aqueous solution was prepared by the following method. 0.45 g of KCl was added to 386.60 g of purified water and dissolved by stirring. 0.45 g of CA was added and dispersed, then the temperature was raised to about 80°C to confirm that it was completely dissolved. 90 g of TC-5M and 22.5 g of TC-5R were added, dispersed, and then defoamed by standing. The temperature was lowered to 50°C, stirred for about 1 hour, and then further stirred at 55°C for about 1 hour. 16.73 g of 22 mass% $TiO_2$ dispersion at 55°C, 3.91 g of 10 mass% KCl aqueous solution at 55°C, and 109.48 g of purified water at 55°C were added, and the solution was stirred for about 15 minutes to obtain the HPMC aqueous solution.

The HPMC aqueous solution containing GE used in examples 38 to 40 was prepared by the following method. 4 g of GE was added to 956 g of purified water and dispersed, then the temperature was raised to about 80°C to confirm that it was completely dissolved. 192 g of TC-5R and 48 g of HPMC with a substitution grade of 2208 (viscosity grade 4.0) were added, dispersed, and then defoamed by standing. The temperature was lowered to 50°C, stirred for about 1 hour, and then further stirred at 55°C for about 1 hour. 1.2 g of 10 mass% KCl aqueous solution at 55°C and 151.2 g of purified water at 55°C were added, and the solution was stirred for about 15 minutes to obtain the HPMC aqueous solution.

3. Pullulan Immersion Solution

1.2 g of KCl was added to 990 g of purified water and dissolved by stirring. 5.1 g of CA was added and dispersed, then the temperature was raised to about 80°C to dissolve CA completely. 204 g of pullulan was added and dissolved by stirring, and then defoamed by standing. The temperature was lowered to 55°C and stirred for about 1 hour obtain a pullulan aqueous solution.

4. PVA Immersion Solution

1.2 g of KCl was added to 953 g of purified water and dissolved by stirring. 240 g of PVA and 6 g of CA were added and dispersed, then the temperature was increased to about 80 °C to confirm that the PVA and CA were completely dissolved, and the solution was defoamed by standing. The temperature was lowered to 55°C and stirred for about 1 hour obtain a PVA aqueous solution.

5. Immersion Solution for Enteric Polymer Outer Layer

An immersion solution for enteric polymer outer layer containing water and anhydrous ethanol as a solvent was prepared by the following method. After mixing 40 g of purified water and 360 g of anhydrous ethanol, 100 g of AS-LG was added to the mixture and dissolved by stirring. After the concentration of the immersion solution for enteric polymer outer layer was adjusted by adding a mixed solvent of purified water and EtOH at the same ratio as described above, it was visually confirmed that the enteric polymer was uniformly dissolved, and used in a molding step. Solutions of AS-MG, AS-HG, HP-50, HP-55, HP-55S and CMEC were prepared by the same procedure.

An immersion solution for enteric polymer outer layer containing water and IPA or acetone as a solvent was prepared by the following method. After mixing 40 g of purified water and 360 g of IPA, 100 g of AS-LG was added and dissolved by stirring. After the concentration of the immersion solution for enteric polymer outer layer was adjusted by adding the mixed solvent at the above ratio, it was visually confirmed that the enteric polymer was uniformly dissolved, and used in the molding step. The immersion solution for the enteric polymer outer layer using acetone as solvent was prepared by the same procedure. Solutions of AS-HG and CMEC were prepared by the same procedure.

An immersion solution for enteric polymer outer layer containing water, anhydrous ethanol and IPA as the solvent was prepared by the following method. After mixing 40 g of purified water, 180 g of EtOH and 180 g of IPA, 100 g of AS-LG was added and dissolved by stirring. After the concentration of the immersion solution for enteric polymer outer layer was adjusted by adding the mixed solvent of purified water and EtOH at the same ratio as described above, it was visually confirmed that the enteric polymer was uniformly dissolved, and used in the molding step.

6. Immersion Solution for Enteric Polymer Outer Layer Containing Shell-Forming Aid

After mixing 40 g of purified water and 360 g of EtOH, 2 g of SLS was added and dissolved by stirring. 40 g of Talc was added and dispersed by stirring, and then 58 g of AS-LG was added and dissolved by stirring. After the concentration of the immersion solution for enteric polymer outer layer was adjusted by adding the mixed solvent of purified water and EtOH at the same ratio as described above, it was visually confirmed that the enteric polymer was uniformly dissolved, and used in the molding step. Solutions of AS-MG, AS-HG, HP-50, HP-55, HP-55S and CMEC were prepared by the same procedure.

7. Immersion Solution for Enteric Polymer Outer Layer Containing Water-Soluble Dye

After mixing 40 g of purified water and 360 g of EtOH, 0.5 g of blue No. 1 or red No. 102 was added and dissolved by stirring. 99.5 g of AS-LG was added and dissolved by stirring. After the concentration of the immersion solution for enteric polymer outer layer was adjusted by adding the mixed solvent of purified water and EtOH at the same ratio as described above, it was visually confirmed that the enteric polymer was uniformly dissolved, and used in the molding step. A solution of AS-HG was prepared by the same procedure.

8. Immersion Solution for Enteric Polymer Outer Layer with Ammonia Solution as Solvent

An immersion solution for enteric polymer outer layer containing AS-LG with ammonia solution as a solvent was prepared by the following method. 70.0 g of AS-LG was added to 506.3 g of purified water, stirred and dispersed, then 7.4 g of ammonia solution was added dropwise, and it was visually confirmed that AS-LG was uniformly dissolved. Purified water was added to the mixture to adjust the viscosity and the mixture was used in the molding step.

An immersion solution for enteric polymer outer layer containing AS-HG with ammonia solution as a solvent was prepared by the following method. 70.0 g of AS-HG was added to 492.7 g of purified water, stirred and dispersed, then 3.9 g of ammonia solution was added dropwise and it was visually confirmed that the AS-HG dissolved uniformly. Purified water was added to the mixture to adjust the viscosity and the mixture was used in the molding step.

9. Representation of Concentration of Immersion Solution for Outer Layer

The concentration of the immersion solution for outer layer consisting only of enteric polymer prepared in the above section 5 is represented as polymer concentration, while the concentration of the immersion solution for outer layer containing shell-forming aid prepared in the above section 6 is represented as solid component concentration.

III. Molding Method of Multilayered Enteric Hard Capsule

A molding method of a multilayered enteric hard capsule to be used in the examples, comparative examples and formulation examples is as described below.

1. Molding Method of Inner Layer Shell

An inner layer of the multilayered enteric hard capsule was prepared by a cold pin immersion method using the gelatine, HPMC, PVA or pullulan immersion solution for the inner layer prepared in the above section II. A mold pin (size No. 2) left at room temperature (about 25°C) was immersed in the immersion solution for the inner layer at about 55°C for a few seconds and then pulled up into the air. The mold pin to which the immersion solution for the inner layer adhered was inverted upside down and dried by blowing air at room ambient temperature for 1 hours or more.

2. Molding Method of Outer Layer Shell

(1) Immersion Solution for Enteric Polymer Outer Layer Described in Above Sections II. 5. to 7.

The mold pin (size No. 2), which was left at room temperature (about 25°C) with the inner layer shell molded in the above sections II. 1. to 4. adhered to it, was immersed in the immersion solution for enteric polymer outer layer prepared in the above section II. for a few seconds and then pulled up into the air. The mold pin to which the immersion solution for the outer layer adhered was inverted upside down and dried by blowing air at room ambient temperature for 1 hours or more.

When the thickness of the outer layer was to be further increased, or when multiple substrates were to be laminated, the molding step was repeated using the immersion solution for enteric polymer outer layer prepared in the above section II.

The shell of the multilayered enteric hard capsule with the outermost layer dried was pulled from the mold pin and cut into predetermined lengths.

(2) Immersion Solution for Enteric Polymer Outer Layer Described in Above Section II. 8.

The mold pin (size No. 2), which was left at room temperature (about 25°C) with the inner layer shell of HPMC molded adhered to it, was immersed in the immersion solution for enteric polymer outer layer prepared in the above section II.8.1. for a few seconds and then pulled up into the air. The mold pin to which the immersion solution for the outer layer adhered was inverted upside down and dried at 30°C/7%RH for 2 hours or more, and then dried at 60°C/6%RH overnight. The shell of the multilayered enteric hard capsule with the outermost layer dried was pulled from the mold pin and cut into predetermined lengths.

IV. Method of Producing Capsule for Evaluation

A method of producing a capsule for evaluation to be used in the examples and comparative examples is as described below.

In the present invention, the solubility (dissolution characteristics) of the capsule itself was evaluated by evaluating dissolution of fast-dissolving acetaminophen. A mixed powder of 20 mass% acetaminophen, 70 mass% lactose, and 10 mass% sodium starch glycolate was prepared, and 200 mg of the mixed powder per capsule was filled into a body, and then a cap was fully fitted to the body to obtain the capsule for evaluation.

When a band seal was further applied, the immersion solution for enteric polymer outer layer, which was used to immerse the outermost layer of the fully fitted capsule, was applied as the band seal to a fitted portion between the cap and body, and air-dried at room ambient temperature to obtain the capsule for evaluation.

V. Method of Determination and Test

1. Dissolution Test of Capsule

In the present invention, the dissolution test described in the Japanese Pharmacopoeia, 18th Edition was applied in principle. However, the Japanese Pharmacopoeia does not specify a solubility of an empty hard capsule itself, so the dissolution test was performed in accordance with the dissolution test method specified in the Japanese Pharmacopoeia (18th Pharmacopoeia, 6.10-1.2 Paddle Method (paddle rotation speed: 50 rpm), with a sinker corresponding to FIG. 6.10-2a being used), to thereby determine a change over time of a dissolution rate of the acetaminophen (AA). A bath-type dissolution tester Model 2100 or Model 2500 manufactured by Distek Inc. was used for the dissolution test. Opt-Diss410 manufactured by Distek Inc. or OD LITE UV FiberOptic available from Leap Technologies was used for absorbance measurement. The absorbance at 244 nm when the same volume of acetaminophen was separately dissolved in an entire amount in a solution in a dissolution tester bath was set to 100%, and a dissolution rate was determined based on the absorbance at 244 nm in the solution in the dissolution tester bath that increased in association with dissolution of the AA from the capsule. The number of samples was set to 1 to 6. The following aqueous solutions were used here as a first fluid, a second fluid, a solution of pH 5.0, and a solution of pH 6.0. In all cases, a temperature of the solutions in the bath was set at $37 \pm 0.5$°C.

2.0 g of sodium chloride was added with 7.0 mL of hydrochloric acid and water to be dissolved, and its volume was adjusted to 1000 mL to obtain the first fluid (pH: about 1.2).

250 mL of a 0.2 mol/L potassium dihydrogen phosphate solution was added with 118 mL of a 0.2 mol/L sodium hydroxide solution and water to be dissolved, and its volume was adjusted to 1000 mL to obtain the second fluid (pH: about 6.8).

The pH 5.0 and pH 6.0 solutions were prepared by adjusting the pH using 0.05 mol/L sodium monohydrogen phosphate and 0.025 mol/L citric acid.

As a decision criterion for results, when the dissolution rate was 1% or more, it was defined as "dissolved", and when the dissolution rate was 80% or more, it was defined as "highly dissolved".

2. Shear Viscosity of Immersion Solution for Enteric Polymer Outer Layer

A shear viscosity of the immersion solution for the enteric outer layer was measured using a rheometer (MCR102) available from Anton Paar Japan K.K. A jig (CC27) and a concentric cylinder tube (CC27/T200/SS) were used for the measurement. The shear viscosity was taken at a temperature of 25°C and a shear rate of 1 sec$^{-1}$.

3. Shell Thicknesses of Inner and Outer Layers

The thickness of the dried shell was measured using an optical micrometer (LS-9030) available from Keyence Corporation. A measurement point for the cap was 5 mm from the top of the mold pin and a measurement point for the body was 8 mm from the top of the mold pin. The shell thicknesses (thickness of a single shell) of the inner and outer layers were calculated by the equation below from measurements of outer diameters of the mold pin, the mold pin to which the inner layer was attached, and the mold pin to which the outer and inner layers were attached. The shell thicknesses of 5 caps and 5 bodies were averaged to obtain an average shell thickness for the capsule of interest.

Equation 1

shell thicknesses of inner layer (μm) = {outer diameter of (inner layer + mold pin) (μm)} − outer diameter of (mold pin) (μm)} / 2

shell thicknesses of outer layer (μm) = {outer diameter of (outer layer + inner layer + mold pin) (μm)} − outer diameter of (inner layer + mold pin) (μm)} / 2

4. Observation of Shell Structure

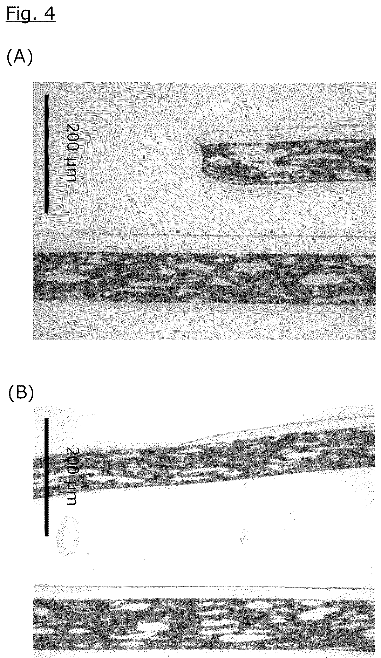

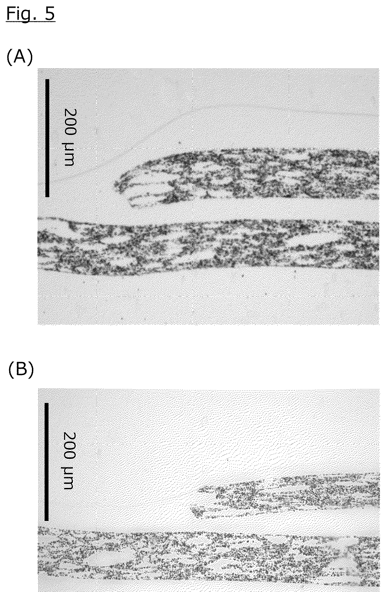

DM5000B manufactured by Leica Microsystems was used as a light microscope. In order to observe a cross section of the capsule shell, the capsule shell in which the body and cap were bonded with epoxy resin was cut into small round slices, embedded in epoxy resin, and then thinly sliced with a microtome to prepare sections for observation (approximately 2000 to 3000 μm square and approximately 10 μm thick). The prepared sections were sandwiched between a microscope slide and a cover glass with oil to prepare samples. The prepared samples were observed under a microscope in bright field by a contrast method. FIGs. 4 and 5 show a show microscopic images without band seal. FIG. 4A corresponds to FIG. 2A. FIG. 4B corresponds to FIG. 2B. FIG. 5A corresponds to FIG. 3A. FIG. 5B corresponds to FIG. 3B.

5. Determination of Ammonia Content

10 mg of sample was taken into a volumetric flask, and 50 mL of pure water was added and extraction was performed by shaking the flask for 10 minutes. Ammonium ions in the extract were measured by ion chromatography and quantified using an absolute calibration curve method. ICS-5000$^+$ manufactured by Thermo Fisher Scientific K.K. was used as an ion chromatograph.

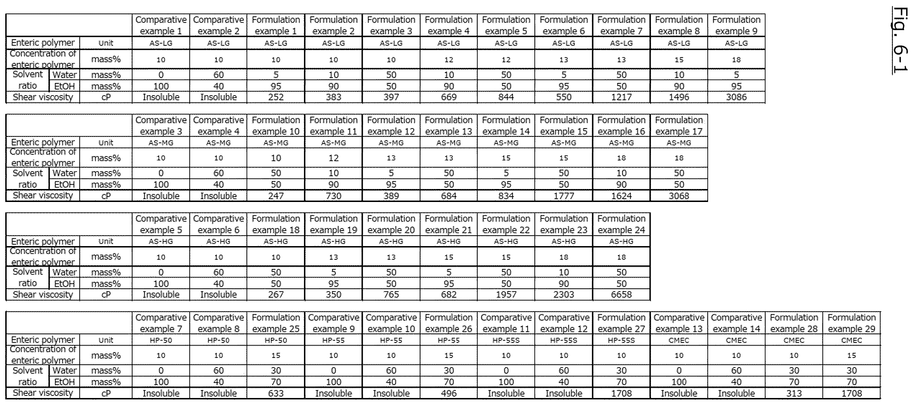

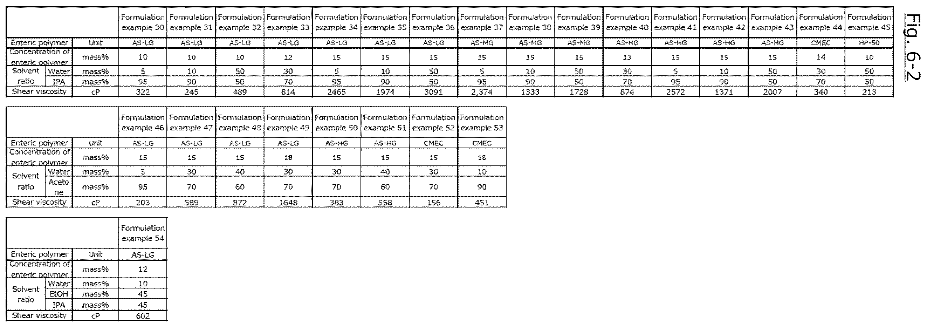

Results of evaluation of the solubility of each enteric polymer in the mixed solvent of water and EtOH are shown in FIG. 6. It was shown that when the EtOH ratio was less than 50 mass% or 96 mass% or more, all of the enteric polymers were insoluble and it was confirmed that they could not be used as the immersion solution for enteric polymer outer layer (comparative examples 1 to 14). On the other hand, when the EtOH ratio was 50 mass% or more and 95 mass% or less, it was shown that all enteric polymers were dissolved, and it was confirmed that the shear viscosities of all the enteric polymers were 150cP or more (formulation examples 1 to 29). In addition, FIG. 6-2 shows results obtained using a mixed solvent of water and IPA, a mixed solvent of water and acetone, or a mixed solvent of water, EtOH, and IPA instead of the mixed solvent of water and EtOH. When the organic solvent ratio was 50 mass% or more and 95 mass% or less, it was shown that all enteric polymers were dissolved, and it was confirmed that the shear viscosities of all enteric polymers were 150cP or more (formulation examples 30 to 54).

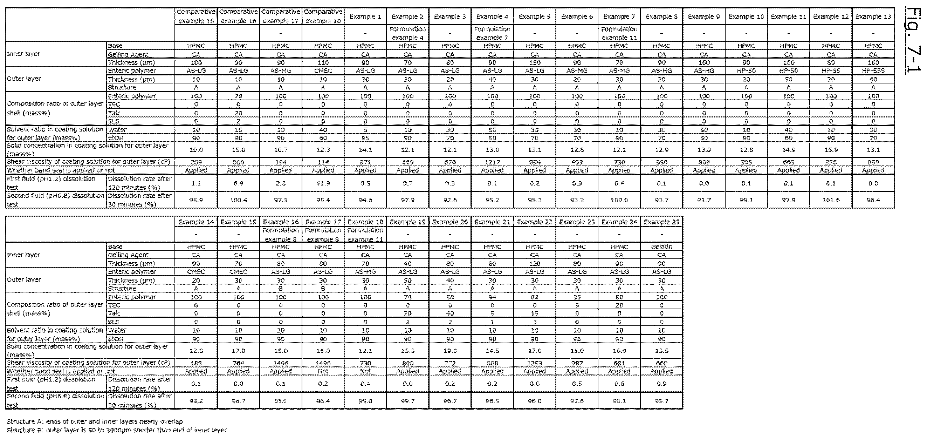

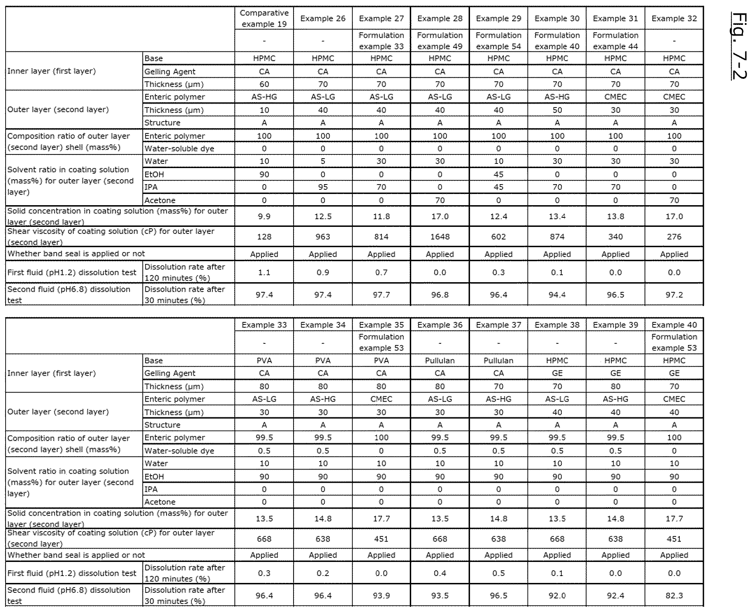

Each of the resulting capsules was then subjected to the dissolution test in the first and second fluids according to the above section V-1. Results obtained when the solvent of the immersion solution for enteric polymer outer layer was the mixed solvent of water and EtOH are shown in FIG. 7. The capsules with an outer layer thickness of 10 μm or less all had a dissolution rate of 1% or more after 2 hours in the first fluid, confirming that they have insufficient acid resistance to acidic solutions (comparative example 15 to 18). On the other hand, the capsules with an outer layer thickness of 20 μm or more had sufficient acid resistance with a dissolution rate of less than 1% after 2 hours in the first fluid and a dissolution rate of 80% or more after 30 minutes in the second fluid, indicating that the capsules were enteric hard capsules having both acid resistance in the acidic range and immediate-release property in the neutral range (examples 1 to 15). In addition, FIG. 7-2 shows a result of a dissolution test of a capsule with an outer layer thickness of 10 $\mu$m or less, using AS-HG as an enteric polymer, as a comparative example19. Even when the enteric polymer was AS-HG, capsules with an outer layer thickness of 10 $\mu$m or less had an dissolution rate of 1% or more after 2 hours in the first fluid, indicating that the capsule does not have sufficient acid resistance to acidic solutions.

Next, an influence of the shell structure was evaluated. Examples 16 and 17, in which a portion of the inner layer was not dip-coated with an enteric outer layer, both had dissolution rates of less than 1% after 2 hours in the first fluid, indicating that they have excellent acid resistance.

Next, an influence whether the band seal was applied or not was evaluated. If the band seal was not applied, there was a concern that the acid solution might penetrate through the fitted portion between the cap and the body. However, when comparing between examples 16 and 17 and between examples 7 and 18, the dissolution rate in the first fluid after 2 hours was all less than 1%, indicating that they have excellent acid resistance regardless whether the band seal is applied or not.

Next, an influence of the shell-forming aid was evaluated. Although examples 19 to 24 contain the shell-forming aid other than the enteric polymer in the outer layer shell, the dissolution rate after 2 hours in the first fluid was all less than 1%, and the dissolution rate after 30 minutes in the second fluid was all 80% or more, indicating no influence on the enteric property.

FIG. 7-2 shows the results of the dissolution tests for each capsule obtained using the immersion solution for enteric polymer outer layer using the mixed solvent of water and IPA, the mixed solvent of water and acetone, or the mixed solvent of water, EtOH, and IPA instead of the mixed solvent of water and EtOH. As shown in examples 26 to 32, all of the capsules with an outer layer thickness of 20 $\mu$m or more had the dissolution rate of less than 1% after 2 hours in the first solution, and all of them had the dissolution rate of 80% or more after 30 minutes in the second solution. This indicates that other hydrophilic organic solvents can be used instead of EtOH.

Next, an influence of the shell component of the inner layer was evaluated. The multilayered enteric hard capsule with the inner layer of gelatine in example 25 shown in FIG. 7 had the dissolution rate of less than 1% after 2 hours in the first fluid and the dissolution rate of 80% or more after 30 minutes in the second fluid.

Also shown in FIG. 7-2 is the multilayered enteric hard capsule in which examples 33 to 35 contain PVA as the base for the inner layer, examples 36 to 37 contain pullulan as the base for the inner layer, and examples 38 to 40 contain HPMC as the base for the inner layer and gellan gum as the gelling agent. All of the multilayered enteric hard capsules of these examples had the dissolution rate of less than 1% after 2 hours in the first fluid, and the dissolution rate of 80% or more after 30 minutes in the second fluid.

These results are similar to other cases where the first layer is HPMC, indicating that the type of substrate contained in the capsule shell of the first layer does not affect the enteric property.

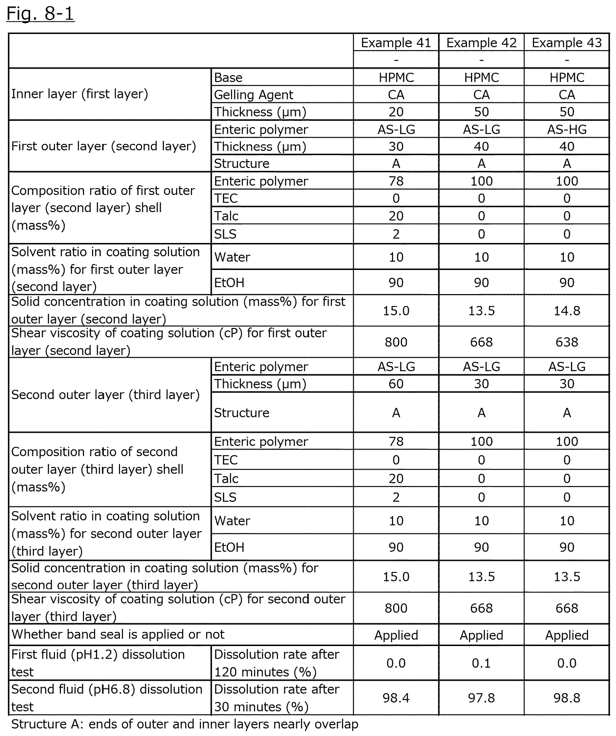

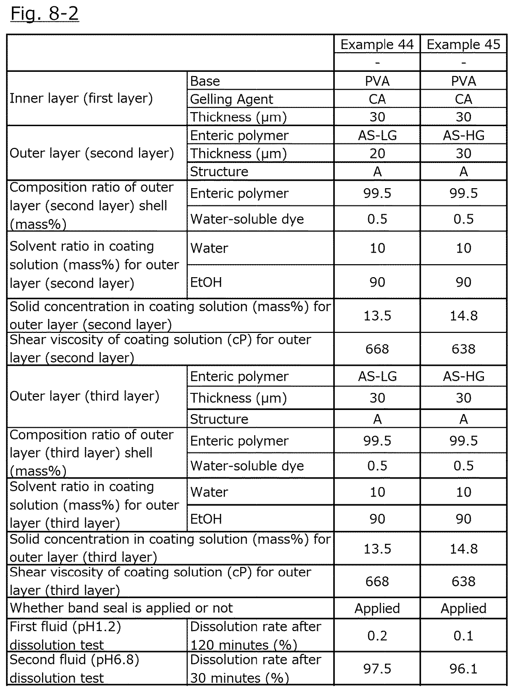

Next, an influence of a three-layer structure was evaluated. In examples 41 to 42 shown in FIG. 8, the shell of the first layer (inner layer) contains HPMC as the base, while the second layer (first outer layer) and the third layer (second outer layer) are molded by repeating the immersion and drying steps twice, using the same immersion solution for the outer layer, respectively. All of the multilayered enteric hard capsules of these examples had the dissolution rate of less than 1% after 2 hours in the first fluid, and the dissolution rate of 80% or more after 30 minutes in the second fluid. indicating that the three-layer structure does not affect the enteric property.

In addition, a hard capsule in which the first outer layer (second layer) and the second outer layer (third layer) contain different types of enteric polymers was evaluated. Example 43 shown in FIG. 8 has a multilayered structure in which the first layer (inner layer) contains HPMC, the second layer (first outer layer) contains HPMCAS-HG, and the third layer (second outer layer) contains HPMCAS-LG. The hard capsule of example 43 had the dissolution rate of less than 1% after 2 hours in the first fluid, and the dissolution rate of 80% or more after 30 minutes in the second fluid. indicating that it has excellent enteric properties.

FIG. 8-2 shows the dissolution rate of three-layered capsules in which the base of the inner layer was replaced by PVA. In examples 44 to 45, the first layer (inner layer) contains PVA as the base, while the second layer (first outer layer) and the third layer (second outer layer) are molded by repeating the immersion and drying steps twice, using the same immersion solution for the outer layer, respectively. All of the multilayered enteric hard capsules of these examples had the dissolution rate of less than 1% after 2 hours in the first fluid, and the dissolution rate of 80% or more after 30 minutes in the second fluid. indicating that the enteric property was not affected.

Next, dissolution tests were performed using solutions other than the first fluid and the second fluid (water (purified water), solution of pH 5.0, and solution of pH 6.0). This test takes into account the fact that capsules are taken with water, for example, the pH in the stomach after eating or drinking increases to about pH 4 to 6. If the dissolution rate increases in this pH range, the capsules may dissolve in the stomach, possibly making postprandial administration difficult.

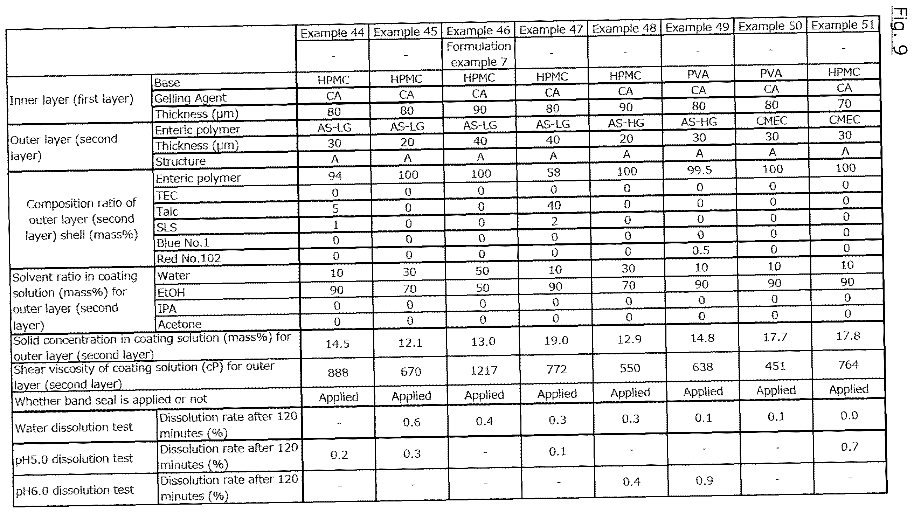

FIG. 9 shows results. The capsules with the outer layer using the immersion solution for enteric polymer outer layer prepared in the above sections II.5 to II.7 (examples 44 to 51) all had a dissolution rate of less than 1% after 2 hours in water, solution of pH 5.0, or solution of pH 6.0, indicating that they had good resistance. These results indicate that there is no difference in resistance whether the enteric polymer contained in the outer layer is AS-LG, CMEC, or AS-HG. Furthermore, it is shown that there is also no difference in the substrate of the inner layer.

One method for dissolving a hydrophobic enteric polymer in a hydrophilic solvent is to use an alkaline solution such as ammonia to neutralize a hydrophobic group of the enteric polymer and dissolve it.

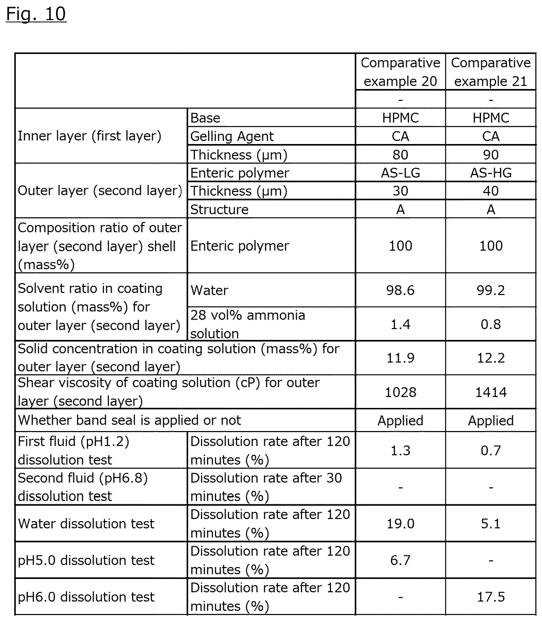

The capsules of comparative examples 20 to 21 shown in FIG. 10, prepared by the method described in the above section III.2.2 (2) using the immersion solution for enteric polymer outer layer described in the above section II.2.8, were subjected to the dissolution test using water, the solution of pH 5.0 and the solution of pH 6.0. The degree of neutralization of the capsules in comparative examples 20 to 21 was calculated from the ammonia content, and about 16 moles out of 100 moles of carboxyl groups of the enteric polymer were neutralized. As a result, it was shown that the dissolution rate in water, the solution of pH5.0, and the solution of pH6.0 solution was higher in the capsule using the immersion solution for enteric polymer outer layer, in which enteric polymer was dissolved by neutralization, for the outer layer than in the capsule using the immersion solution for enteric polymer outer layer prepared in the above sections II.5 to II.7 for the outer layer. These results indicate that alkaline neutralization impairs an acid-resistant function of the enteric polymers such as AS-LG and AS-HG.

Accordingly, it was found that capsule using the immersion solution for enteric polymer outer layer prepared in the above sections II.5 to II.7 for the outer layer can more reliably release the filling in the capsule into an intestine considering the actual situation of taking the capsule.

A concentration of ammonium ion ($NH4^+$) in the shell (including inner and outer layers) was measured by the following method.

10 mg of the shell was taken in the volumetric flask, 50 mL of pure water was added, and the extraction was performed by shaking the flask for 10 minutes. $NH4^+$ in the extract was measured by the ion chromatography and quantified by the absolute calibration curve method from the actual peak intensity. ICS-5000$^+$ manufactured by Thermo Fisher Scientific K.K. was used as the ion chromatograph. A lower detection limit of this measurement system is 0.0010 g (10 ppm).

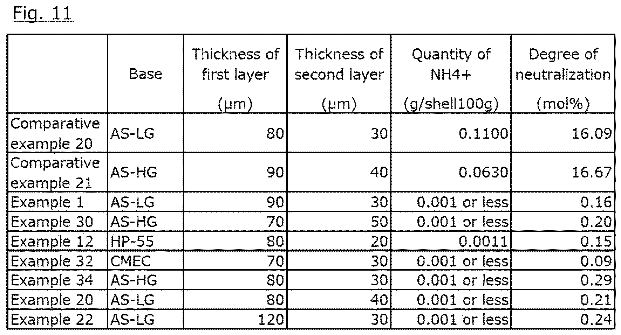

FIG. 11 shows the content of ammonium ions in 100 g of the shell and the degree of neutralization for comparative examples 20 and 21 and examples 1, 30, 12, 32, 34, 20, and 22. The 100 g of the shell includes the inner and outer layers. The content of ammonium ions per 100 g of shell was 0.1100 g (1,100 ppm) for comparative example 20 and 0.0630 g (630 ppm) for comparative example 21. On the other hand, in the examples, the content of ammonium ions per 100 g of the shell was 0.0011 g (11 ppm) or not more than 0.0010 g (10 ppm). Since no ammonium ions or compounds that may generate ammonium ions were intentionally added in the examples, if at least 0.0015 g (15 ppm) or less of ammonium ions were present, it is considered that the shell is substantially free of ammonium ions.

The degree of neutralization of the carbocyclic groups of the base in the shell was 16.09 mol% for comparative example 20 and 16.67 mol% for comparative example 21. On the other hand, in the examples, the degree of neutralization of the carbocyclic groups of the base in the shell ranged from 0.09 to 0.29 mol%.

**Claims**

1. A multilayered enteric hard capsule comprising a cap portion and a body portion, the cap portion and the body portion each consisting of a capsule shell comprising at least a first layer and a second layer in whole or in part, wherein the first layer contains a water-soluble base, the second layer contains an enteric polymer, and the second layer coats an outside of the first layer in whole or in part.

2. The multilayered enteric hard capsule according to claim 1, wherein a thickness of the first layer of the capsule shell is 20 to 160 $\mu$m and a thickness of the second layer of the capsule shell is 20 to 70 $\mu$m.

3. The multilayered enteric hard capsule according to claim 2, wherein the combined thickness of the first and second layers of the capsule shell is 70 to 150 $\mu$m.

4. The multilayered enteric hard capsule according to claim 1, consisting of the second layer containing

    (1) an enteric polymer whose content is 50 mass% or more when the total content of the shell components excluding moisture is set to 100 mass%, and
    (2) a shell-forming aid.

5. The multilayered enteric hard capsule according to claim 4, wherein the enteric polymer is one selected from the group consisting of hydroxypropyl methylcellulose acetate succinate, hydroxypropyl methylcellulose phthalate, carboxymethyl ethyl cellulose, and mixtures thereof.

6. The multilayered enteric hard capsule according to claim 4, wherein percentage of acid residues neutralized in the enteric polymer in the capsule shell is 0.01% or less when the number of moles (number of groups) of acid residues in the enteric polymer before neutralization is set to 100%.

7. The multilayered enteric hard capsule according to claim 4, wherein the shell-forming aid is at least one selected from the group consisting of a plasticizer, a surfactant, and a surface modifier.

8. The multilayered enteric hard capsule according to claim 7, wherein the shell-forming aid is at least one selected from the group consisting of triethyl citrate, talc and sodium lauryl sulphate.

9. The multilayered enteric hard capsule according to claim 1, wherein the water-soluble base is hydroxypropyl methylcellulose, gelatine, pullulan, or polyvinyl alcohol.

10. The multilayered enteric hard capsule according to claim 1, wherein a dissolution rate of the content filled in the capsule is less than 1% after 2 hours in a dissolution test using a solution with pH 1.2.

11. The multilayered enteric hard capsule according to claim 10, wherein the dissolution rate of the content filled in the capsule is less than 1% after 2 hours in the dissolution test using a solution with pH 5.0 or 6.0.

12. The multilayered enteric hard capsule according to claim 10, wherein the dissolution rate of the content filled in the capsule is 80% or more after 30 minutes in the dissolution test using a solution with pH 6.8.

13. The multilayered enteric hard capsule according to claim 1, wherein

    the dissolution rate of the content filled in the capsule is less than 1% after 2 hours in the dissolution test using the solution with pH 1.2,
    the dissolution rate of the content filled in the capsule is less than 1% after 2 hours in the dissolution test using the solution with pH 5.0 or 6.0, and
    the dissolution rate of the content filled in the capsule is 80% or more after 30 minutes in the dissolution test using the solution with pH 6.8.

14. The multilayered enteric hard capsule according to claim 1, wherein the content of the neutralizer per 100 g of capsule shell is 0.06 g or less.

15. An immersion solution for second layer for forming the second layer of the multilayered enteric hard capsule according to claim 1, wherein an enteric polymer, or a solid component containing the enteric polymer and a shell-forming aid, is dissolved or dispersed in a mixed solvent of water and a hydrophilic organic solvent, the ratio of anhydrous ethanol in the mixed solvent being 50 mass% or more and 95 mass% or less, and a shear viscosity of the immersion solution for second layer being 150 cP or more at 25°C.

16. The immersion solution for second layer according to claim 15, wherein a concentration of the enteric polymer in the immersion solution for second layer is 10 mass% or more.

17. The immersion solution for second layer according to claim 15, wherein the content of the neutralizer in the immersion solution for second layer is 0.01 mass% or less when a total amount of the immersion solution for second layer is set to 100 mass%.

18. The immersion solution for second layer according to claim 15, wherein the hydrophilic organic solvent is at least one selected from the group consisting of anhydrous ethanol, 2-propanol, and acetone.

19. A method of producing a multilayered enteric hard capsule comprising:

    a step of preparing a hard capsule for a first layer of a multilayered enteric hard capsule; and
    a step of immersing the hard capsule for the first layer in the immersion solution for second layer according to claim 15, taking the hard capsule for the first layer out of the immersion solution for second layer, removing a mixed solvent in the immersion solution for second layer adhered to the hard capsule for the first layer, and molding the second layer of the hard capsule for the multilayered enteric hard capsule.

20. The method according to claim 19, wherein the step of molding the second layer is a plurality of times.

Fig. 1

## Fig. 2

(A)

11 — cap – outer layer

15 —

cap – inner layer

21 —

12

body – outer layer

body – inner layer

25

22

3

(B)

11 —

cap – outer layer

15 —

cap – inner layer

21 —

12

body – outer layer

body – inner layer

25

22

3

(C)

11 —

cap – outer layer

15 —

cap – inner layer

21 —

12

body – outer layer

body – inner layer

25

22

3

Fig. 3

(A)

11

15
band seal

21

cap – outer layer

cap – inner layer

body – outer layer

body – inner layer

12

25

22

3

(B)

11

15
band seal

21

cap – outer layer

cap – inner layer

body – outer layer

body – inner layer

12

25

22

3

Fig. 4

(A)

(B)

Fig. 5

(A)

(B)

Fig. 6-1

EP 4 559 458 A1

|  |  | Comparative example 1 | Comparative example 2 | Formulation example 1 | Formulation example 2 | Formulation example 3 | Formulation example 4 | Formulation example 5 | Formulation example 6 | Formulation example 7 | Formulation example 8 | Formulation example 9 |
|---|---|---|---|---|---|---|---|---|---|---|---|---|
| Enteric polymer | Unit | AS-LG | AS-LG | AS-LG | AS-LG | AS-LG | AS-LG | AS-LG | AS-LG | AS-LG | AS-LG | AS-LG |
| Concentration of enteric polymer | mass% | 10 | 10 | 10 | 10 | 10 | 12 | 12 | 13 | 13 | 15 | 18 |
| Solvent ratio | Water mass% | 0 | 60 | 5 | 10 | 50 | 10 | 50 | 5 | 50 | 10 | 5 |
|  | EtOH mass% | 100 | 40 | 95 | 90 | 50 | 90 | 50 | 95 | 50 | 90 | 95 |
| Shear viscosity | cP | Insoluble | Insoluble | 252 | 383 | 397 | 669 | 844 | 550 | 1217 | 1496 | 3086 |

|  |  | Comparative example 3 | Comparative example 4 | Formulation example 10 | Formulation example 11 | Formulation example 12 | Formulation example 13 | Formulation example 14 | Formulation example 15 | Formulation example 16 | Formulation example 17 |
|---|---|---|---|---|---|---|---|---|---|---|---|
| Enteric polymer | Unit | AS-MG | AS-MG | AS-MG | AS-MG | AS-MG | AS-MG | AS-MG | AS-MG | AS-MG | AS-MG |
| Concentration of enteric polymer | mass% | 10 | 10 | 10 | 12 | 13 | 13 | 15 | 15 | 18 | 18 |
| Solvent ratio | Water mass% | 0 | 60 | 50 | 10 | 5 | 50 | 5 | 50 | 10 | 50 |
|  | EtOH mass% | 100 | 40 | 50 | 90 | 95 | 50 | 95 | 50 | 90 | 50 |
| Shear viscosity | cP | Insoluble | Insoluble | 247 | 730 | 389 | 684 | 834 | 1777 | 1624 | 3068 |

|  |  | Comparative example 5 | Comparative example 6 | Formulation example 18 | Formulation example 19 | Formulation example 20 | Formulation example 21 | Formulation example 22 | Formulation example 23 | Formulation example 24 |
|---|---|---|---|---|---|---|---|---|---|---|
| Enteric polymer | Unit | AS-HG | AS-HG | AS-HG | AS-HG | AS-HG | AS-HG | AS-HG | AS-HG | AS-HG |
| Concentration of enteric polymer | mass% | 10 | 10 | 10 | 13 | 13 | 15 | 15 | 18 | 18 |
| Solvent ratio | Water mass% | 0 | 60 | 50 | 5 | 50 | 5 | 50 | 10 | 50 |
|  | EtOH mass% | 100 | 40 | 50 | 95 | 50 | 95 | 50 | 90 | 50 |
| Shear viscosity | cP | Insoluble | Insoluble | 267 | 350 | 765 | 682 | 1957 | 2303 | 6658 |

|  |  | Comparative example 7 | Comparative example 8 | Formulation example 25 | Comparative example 9 | Comparative example 10 | Formulation example 26 | Comparative example 11 | Comparative example 12 | Formulation example 27 | Comparative example 13 | Comparative example 14 | Formulation example 28 | Formulation example 29 |
|---|---|---|---|---|---|---|---|---|---|---|---|---|---|---|
| Enteric polymer | Unit | HP-50 | HP-50 | HP-50 | HP-55 | HP-55 | HP-55 | HP-55S | HP-55S | HP-55S | CMEC | CMEC | CMEC | CMEC |
| Concentration of enteric polymer | mass% | 10 | 10 | 15 | 10 | 10 | 15 | 10 | 10 | 10 | 10 | 10 | 10 | 15 |
| Solvent ratio | Water mass% | 0 | 60 | 30 | 0 | 60 | 30 | 0 | 60 | 30 | 0 | 60 | 30 | 30 |
|  | EtOH mass% | 100 | 40 | 70 | 100 | 40 | 70 | 100 | 40 | 70 | 100 | 40 | 70 | 70 |
| Shear viscosity | cP | Insoluble | Insoluble | 633 | Insoluble | Insoluble | 496 | Insoluble | Insoluble | 1708 | Insoluble | Insoluble | 313 | 1708 |

Fig. 6-2

| | | Formulation example 30 | Formulation example 31 | Formulation example 32 | Formulation example 33 | Formulation example 34 | Formulation example 35 | Formulation example 36 | Formulation example 37 | Formulation example 38 | Formulation example 39 | Formulation example 40 | Formulation example 41 | Formulation example 42 | Formulation example 43 | Formulation example 44 | Formulation example 45 |
|---|---|---|---|---|---|---|---|---|---|---|---|---|---|---|---|---|---|
| Enteric polymer | Unit | AS-LG | AS-LG | AS-LG | AS-LG | AS-LG | AS-LG | AS-LG | AS-MG | AS-MG | AS-MG | AS-HG | AS-HG | AS-HG | AS-HG | CMEC | HP-50 |
| Concentration of enteric polymer | mass% | 10 | 10 | 10 | 12 | 15 | 15 | 15 | 15 | 15 | 15 | 13 | 15 | 15 | 15 | 14 | 10 |
| Solvent ratio | Water | mass% | 5 | 10 | 50 | 30 | 5 | 10 | 50 | 5 | 10 | 50 | 30 | 5 | 10 | 50 | 30 | 50 |
| | IPA | mass% | 95 | 90 | 50 | 70 | 95 | 90 | 50 | 95 | 90 | 50 | 70 | 95 | 90 | 50 | 70 | 50 |
| Shear viscosity | cP | 322 | 245 | 489 | 814 | 2465 | 1974 | 3091 | 2,374 | 1333 | 1728 | 874 | 2572 | 1371 | 2007 | 340 | 213 |

| | | Formulation example 46 | Formulation example 47 | Formulation example 48 | Formulation example 49 | Formulation example 50 | Formulation example 51 | Formulation example 52 | Formulation example 53 |
|---|---|---|---|---|---|---|---|---|---|
| Enteric polymer | Unit | AS-LG | AS-LG | AS-LG | AS-LG | AS-HG | AS-HG | CMEC | CMEC |
| Concentration of enteric polymer | mass% | 15 | 15 | 15 | 18 | 15 | 15 | 15 | 18 |
| Solvent ratio | Water | mass% | 5 | 30 | 40 | 30 | 30 | 40 | 30 | 10 |
| | Acetone | mass% | 95 | 70 | 60 | 70 | 70 | 60 | 70 | 90 |
| Shear viscosity | cP | 203 | 589 | 872 | 1648 | 383 | 558 | 156 | 451 |

| | | Formulation example 54 |
|---|---|---|
| Enteric polymer | Unit | AS-LG |
| Concentration of enteric polymer | mass% | 12 |
| Solvent ratio | Water | mass% | 10 |
| | EtOH | mass% | 45 |
| | IPA | mass% | 45 |
| Shear viscosity | cP | 602 |

## Fig. 7-1

| | | Comparative example 15 | Comparative example 16 | Comparative example 17 | Comparative example 18 | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|---|---|---|
| | | - | - | - | - | - | Formulation example 4 | - | Formulation example 7 | - | - | Formulation example 11 | - | - | - | - | - | - |
| Inner layer | Base | HPMC | HPMC | HPMC | HPMC | HPMC | HPMC | HPMC | HPMC | HPMC | HPMC | HPMC | HPMC | HPMC | HPMC | HPMC | HPMC | HPMC |
| | Gelling Agent | CA | CA | CA | CA | CA | CA | CA | CA | CA | CA | CA | CA | CA | CA | CA | CA | CA |
| | Thickness (μm) | 100 | 70 | 90 | 110 | 90 | 70 | 80 | 90 | 150 | 90 | 70 | 90 | 160 | 90 | 160 | 80 | 160 |
| Outer layer | Enteric polymer | AS-LG | AS-LG | AS-MG | CMEC | AS-LG | AS-LG | AS-LG | AS-LG | AS-LG | AS-MG | AS-MG | AS-HG | AS-HG | HP-50 | HP-50 | HP-55 | HP-55S |
| | Thickness (μm) | 10 | 10 | 10 | 10 | 30 | 30 | 20 | 40 | 30 | 20 | 30 | 20 | 30 | 20 | 30 | 20 | 40 |
| | Structure | A | A | A | A | A | A | A | A | A | A | A | A | A | A | A | A | A |
| Composition ratio of outer layer shell (mass%) | Enteric polymer | 100 | 78 | 100 | 100 | 100 | 100 | 100 | 100 | 100 | 100 | 100 | 100 | 100 | 100 | 100 | 100 | 100 |
| | TEC | 0 | 0 | 0 | 0 | 0 | 0 | 0 | 0 | 0 | 0 | 0 | 0 | 0 | 0 | 0 | 0 | 0 |
| | Talc | 0 | 20 | 0 | 0 | 0 | 0 | 0 | 0 | 0 | 0 | 0 | 0 | 0 | 0 | 0 | 0 | 0 |
| | SLS | 0 | 2 | 0 | 0 | 0 | 0 | 0 | 0 | 0 | 0 | 0 | 0 | 0 | 0 | 0 | 0 | 0 |
| Solvent ratio in coating solution for outer layer (mass%) | Water | 10 | 10 | 10 | 40 | 5 | 10 | 30 | 50 | 30 | 30 | 10 | 30 | 50 | 10 | 40 | 10 | 30 |
| | EtOH | 90 | 90 | 90 | 60 | 95 | 90 | 70 | 50 | 70 | 70 | 90 | 70 | 50 | 90 | 60 | 90 | 70 |
| Solid concentration in coating solution for outer layer (mass%) | | 10.0 | 15.0 | 10.7 | 12.3 | 14.1 | 12.1 | 12.1 | 13.0 | 13.1 | 12.8 | 12.1 | 12.9 | 13.0 | 12.8 | 14.9 | 15.9 | 13.1 |
| Shear viscosity of coating solution for outer layer (cP) | | 209 | 800 | 194 | 114 | 871 | 669 | 670 | 1217 | 854 | 493 | 730 | 550 | 809 | 505 | 665 | 358 | 859 |
| Whether band seal is applied or not | | Applied | Applied | Applied | Applied | Applied | Applied | Applied | Applied | Applied | Applied | Applied | Applied | Applied | Applied | Applied | Applied | Applied |
| First fluid (pH1.2) dissolution test | Dissolution rate after 120 minutes (%) | 1.1 | 6.4 | 2.8 | 41.9 | 0.5 | 0.7 | 0.3 | 0.1 | 0.2 | 0.9 | 0.4 | 0.1 | 0.0 | 0.1 | 0.1 | 0.1 | 0.0 |
| Second fluid (pH6.8) dissolution test | Dissolution rate after 30 minutes (%) | 95.9 | 100.4 | 97.5 | 95.4 | 94.6 | 97.9 | 92.6 | 95.2 | 95.3 | 93.2 | 100.0 | 93.7 | 91.7 | 99.1 | 97.9 | 101.6 | 96.4 |

| | | Example 14 | Example 15 | Example 16 | Example 17 | Example 18 | Example 19 | Example 20 | Example 21 | Example 22 | Example 23 | Example 24 | Example 25 |
|---|---|---|---|---|---|---|---|---|---|---|---|---|---|
| | | - | - | Formulation example 8 | Formulation example 8 | Formulation example 11 | - | - | - | - | - | - | - |
| Inner layer | Base | HPMC | HPMC | HPMC | HPMC | HPMC | HPMC | HPMC | HPMC | HPMC | HPMC | HPMC | Gelatin |
| | Gelling Agent | CA | CA | CA | CA | CA | CA | CA | CA | CA | CA | CA | CA |
| | Thickness (μm) | 90 | 70 | 80 | 80 | 70 | 40 | 80 | 80 | 120 | 80 | 90 | 90 |
| Outer layer | Enteric polymer | CMEC | CMEC | AS-LG | AS-LG | AS-MG | AS-LG | AS-LG | AS-LG | AS-LG | AS-LG | AS-LG | AS-LG |
| | Thickness (μm) | 20 | 30 | 30 | 30 | 30 | 50 | 40 | 30 | 30 | 30 | 30 | 30 |
| | Structure | A | A | B | B | A | A | A | A | A | A | A | A |
| Composition ratio of outer layer shell (mass%) | Enteric polymer | 100 | 100 | 100 | 100 | 100 | 78 | 58 | 94 | 82 | 95 | 80 | 100 |
| | TEC | 0 | 0 | 0 | 0 | 0 | 0 | 0 | 0 | 0 | 0 | 0 | 0 |
| | Talc | 0 | 0 | 0 | 0 | 0 | 20 | 40 | 5 | 15 | 5 | 20 | 0 |
| | SLS | 0 | 0 | 0 | 0 | 0 | 2 | 2 | 1 | 3 | 0 | 0 | 0 |
| Solvent ratio in coating solution for outer layer (mass%) | Water | 10 | 10 | 10 | 10 | 10 | 10 | 10 | 10 | 10 | 10 | 10 | 10 |
| | EtOH | 90 | 90 | 90 | 90 | 90 | 90 | 90 | 90 | 90 | 90 | 90 | 90 |
| Solid concentration in coating solution for outer layer (mass%) | | 12.8 | 17.8 | 15.0 | 15.0 | 12.1 | 15.0 | 19.0 | 14.5 | 17.0 | 15.0 | 16.0 | 13.5 |
| Shear viscosity of coating solution for outer layer (cP) | | 188 | 764 | 1496 | 1496 | 730 | 800 | 772 | 888 | 1253 | 987 | 681 | 668 |
| Whether band seal is applied or not | | Applied | Applied | Applied | Not | Not | Applied | Applied | Applied | Applied | Applied | Applied | Applied |
| First fluid (pH1.2) dissolution test | Dissolution rate after 120 minutes (%) | 0.1 | 0.0 | 0.1 | 0.2 | 0.4 | 0.0 | 0.2 | 0.2 | 0.0 | 0.5 | 0.6 | 0.9 |
| Second fluid (pH6.8) dissolution test | Dissolution rate after 30 minutes (%) | 93.2 | 96.7 | 95.0 | 96.4 | 95.8 | 99.7 | 96.7 | 96.5 | 96.0 | 97.6 | 98.1 | 95.7 |

Structure A: ends of outer and inner layers nearly overlap
Structure B: outer layer is 50 to 3000μm shorter than end of inner layer

31

Fig. 7-2

| | | Comparative example 19 | Example 26 | Example 27 | Example 28 | Example 29 | Example 30 | Example 31 | Example 32 |
|---|---|---|---|---|---|---|---|---|---|
| | | - | - | Formulation example 33 | Formulation example 49 | Formulation example 54 | Formulation example 40 | Formulation example 44 | - |
| Inner layer (first layer) | Base | HPMC | HPMC | HPMC | HPMC | HPMC | HPMC | HPMC | HPMC |
| | Gelling Agent | CA | CA | CA | CA | CA | CA | CA | CA |
| | Thickness (µm) | 60 | 70 | 70 | 70 | 70 | 70 | 70 | 70 |
| Outer layer (second layer) | Enteric polymer | AS-HG | AS-LG | AS-LG | AS-LG | AS-LG | AS-HG | CMEC | CMEC |
| | Thickness (µm) | 10 | 40 | 40 | 40 | 40 | 50 | 30 | 30 |
| | Structure | A | A | A | A | A | A | A | A |
| Composition ratio of outer layer (second layer) shell (mass%) | Enteric polymer | 100 | 100 | 100 | 100 | 100 | 100 | 100 | 100 |
| | Water-soluble dye | 0 | 0 | 0 | 0 | 0 | 0 | 0 | 0 |
| Solvent ratio in coating solution (mass%) for outer layer (second layer) | Water | 10 | 5 | 30 | 30 | 10 | 30 | 30 | 30 |
| | EtOH | 90 | 0 | 0 | 0 | 45 | 0 | 0 | 0 |
| | IPA | 0 | 95 | 70 | 0 | 45 | 70 | 70 | 0 |
| | Acetone | 0 | 0 | 0 | 70 | 0 | 0 | 0 | 70 |
| Solid concentration in coating solution (mass%) for outer layer (second layer) | | 9.9 | 12.5 | 11.8 | 17.0 | 12.4 | 13.4 | 13.8 | 17.0 |
| Shear viscosity of coating solution (cP) for outer layer (second layer) | | 128 | 963 | 814 | 1648 | 602 | 874 | 340 | 276 |
| Whether band seal is applied or not | | Applied | Applied | Applied | Applied | Applied | Applied | Applied | Applied |
| First fluid (pH1.2) dissolution test | Dissolution rate after 120 minutes (%) | 1.1 | 0.9 | 0.7 | 0.0 | 0.3 | 0.1 | 0.0 | 0.0 |
| Second fluid (pH6.8) dissolution test | Dissolution rate after 30 minutes (%) | 97.4 | 97.4 | 97.7 | 96.8 | 96.4 | 94.4 | 96.5 | 97.2 |

| | | Example 33 | Example 34 | Example 35 | Example 36 | Example 37 | Example 38 | Example 39 | Example 40 |
|---|---|---|---|---|---|---|---|---|---|
| | | - | - | Formulation example 53 | - | - | - | - | Formulation example 53 |
| Inner layer (first layer) | Base | PVA | PVA | PVA | Pullulan | Pullulan | HPMC | HPMC | HPMC |
| | Gelling Agent | CA | CA | CA | CA | CA | GE | GE | GE |
| | Thickness (µm) | 80 | 80 | 80 | 80 | 70 | 70 | 80 | 70 |
| Outer layer (second layer) | Enteric polymer | AS-LG | AS-HG | CMEC | AS-LG | AS-HG | AS-LG | AS-HG | CMEC |
| | Thickness (µm) | 30 | 30 | 30 | 30 | 30 | 40 | 40 | 40 |
| | Structure | A | A | A | A | A | A | A | A |
| Composition ratio of outer layer (second layer) shell (mass%) | Enteric polymer | 99.5 | 99.5 | 100 | 99.5 | 99.5 | 99.5 | 99.5 | 100 |
| | Water-soluble dye | 0.5 | 0.5 | 0 | 0.5 | 0.5 | 0.5 | 0.5 | 0 |
| Solvent ratio in coating solution (mass%) for outer layer (second layer) | Water | 10 | 10 | 10 | 10 | 10 | 10 | 10 | 10 |
| | EtOH | 90 | 90 | 90 | 90 | 90 | 90 | 90 | 90 |
| | IPA | 0 | 0 | 0 | 0 | 0 | 0 | 0 | 0 |
| | Acetone | 0 | 0 | 0 | 0 | 0 | 0 | 0 | 0 |
| Solid concentration in coating solution (mass%) for outer layer (second layer) | | 13.5 | 14.8 | 17.7 | 13.5 | 14.8 | 13.5 | 14.8 | 17.7 |
| Shear viscosity of coating solution (cP) for outer layer (second layer) | | 668 | 638 | 451 | 668 | 638 | 668 | 638 | 451 |
| Whether band seal is applied or not | | Applied | Applied | Applied | Applied | Applied | Applied | Applied | Applied |
| First fluid (pH1.2) dissolution test | Dissolution rate after 120 minutes (%) | 0.3 | 0.2 | 0.0 | 0.4 | 0.5 | 0.1 | 0.0 | 0.0 |
| Second fluid (pH6.8) dissolution test | Dissolution rate after 30 minutes (%) | 96.4 | 96.4 | 93.9 | 93.5 | 96.5 | 92.0 | 92.4 | 82.3 |

## Fig. 8-1

| | | Example 41 | Example 42 | Example 43 |
|---|---|---|---|---|
| | | - | - | - |
| Inner layer (first layer) | Base | HPMC | HPMC | HPMC |
| | Gelling Agent | CA | CA | CA |
| | Thickness (μm) | 20 | 50 | 50 |
| First outer layer (second layer) | Enteric polymer | AS-LG | AS-LG | AS-HG |
| | Thickness (μm) | 30 | 40 | 40 |
| | Structure | A | A | A |
| Composition ratio of first outer layer (second layer) shell (mass%) | Enteric polymer | 78 | 100 | 100 |
| | TEC | 0 | 0 | 0 |
| | Talc | 20 | 0 | 0 |
| | SLS | 2 | 0 | 0 |
| Solvent ratio in coating solution (mass%) for first outer layer (second layer) | Water | 10 | 10 | 10 |
| | EtOH | 90 | 90 | 90 |
| Solid concentration in coating solution (mass%) for first outer layer (second layer) | | 15.0 | 13.5 | 14.8 |
| Shear viscosity of coating solution (cP) for first outer layer (second layer) | | 800 | 668 | 638 |
| Second outer layer (third layer) | Enteric polymer | AS-LG | AS-LG | AS-LG |
| | Thickness (μm) | 60 | 30 | 30 |
| | Structure | A | A | A |
| Composition ratio of second outer layer (third layer) shell (mass%) | Enteric polymer | 78 | 100 | 100 |
| | TEC | 0 | 0 | 0 |
| | Talc | 20 | 0 | 0 |
| | SLS | 2 | 0 | 0 |
| Solvent ratio in coating solution (mass%) for second outer layer (third layer) | Water | 10 | 10 | 10 |
| | EtOH | 90 | 90 | 90 |
| Solid concentration in coating solution (mass%) for second outer layer (third layer) | | 15.0 | 13.5 | 13.5 |
| Shear viscosity of coating solution (cP) for second outer layer (third layer) | | 800 | 668 | 668 |
| Whether band seal is applied or not | | Applied | Applied | Applied |
| First fluid (pH1.2) dissolution test | Dissolution rate after 120 minutes (%) | 0.0 | 0.1 | 0.0 |
| Second fluid (pH6.8) dissolution test | Dissolution rate after 30 minutes (%) | 98.4 | 97.8 | 98.8 |

Structure A: ends of outer and inner layers nearly overlap

Fig. 8-2

| | | Example 44 | Example 45 |
|---|---|---|---|
| | | - | - |
| Inner layer (first layer) | Base | PVA | PVA |
| | Gelling Agent | CA | CA |
| | Thickness (μm) | 30 | 30 |
| Outer layer (second layer) | Enteric polymer | AS-LG | AS-HG |
| | Thickness (μm) | 20 | 30 |
| | Structure | A | A |
| Composition ratio of outer layer (second layer) shell (mass%) | Enteric polymer | 99.5 | 99.5 |
| | Water-soluble dye | 0.5 | 0.5 |
| Solvent ratio in coating solution (mass%) for outer layer (second layer) | Water | 10 | 10 |
| | EtOH | 90 | 90 |
| Solid concentration in coating solution (mass%) for outer layer (second layer) | | 13.5 | 14.8 |
| Shear viscosity of coating solution (cP) for outer layer (second layer) | | 668 | 638 |
| Outer layer (third layer) | Enteric polymer | AS-LG | AS-HG |
| | Thickness (μm) | 30 | 30 |
| | Structure | A | A |
| Composition ratio of outer layer (third layer) shell (mass%) | Enteric polymer | 99.5 | 99.5 |
| | Water-soluble dye | 0.5 | 0.5 |
| Solvent ratio in coating solution (mass%) for outer layer (third layer) | Water | 10 | 10 |
| | EtOH | 90 | 90 |
| Solid concentration in coating solution (mass%) for outer layer (third layer) | | 13.5 | 14.8 |
| Shear viscosity of coating solution (cP) for outer layer (third layer) | | 668 | 638 |
| Whether band seal is applied or not | | Applied | Applied |
| First fluid (pH1.2) dissolution test | Dissolution rate after 120 minutes (%) | 0.2 | 0.1 |
| Second fluid (pH6.8) dissolution test | Dissolution rate after 30 minutes (%) | 97.5 | 96.1 |

Fig. 9

| | | Example 44 | Example 45 | Example 46 | Example 47 | Example 48 | Example 49 | Example 50 | Example 51 |
|---|---|---|---|---|---|---|---|---|---|
| | | - | - | Formulation example 7 | - | - | - | - | - |
| Inner layer (first layer) | Base | HPMC | HPMC | HPMC | HPMC | HPMC | PVA | PVA | HPMC |
| | Gelling Agent | CA | CA | CA | CA | CA | CA | CA | CA |
| | Thickness (μm) | 80 | 80 | 90 | 80 | 90 | 80 | 80 | 70 |
| Outer layer (second layer) | Enteric polymer | AS-LG | AS-LG | AS-LG | AS-LG | AS-HG | AS-HG | CMEC | CMEC |
| | Thickness (μm) | 30 | 20 | 40 | 40 | 20 | 30 | 30 | 30 |
| | Structure | A | A | A | A | A | A | A | A |
| Composition ratio of outer layer (second layer) shell (mass%) | Enteric polymer | 94 | 100 | 100 | 58 | 100 | 99.5 | 100 | 100 |
| | TEC | 0 | 0 | 0 | 0 | 0 | 0 | 0 | 0 |
| | Talc | 5 | 0 | 0 | 40 | 0 | 0 | 0 | 0 |
| | SLS | 1 | 0 | 0 | 2 | 0 | 0 | 0 | 0 |
| | Blue No.1 | 0 | 0 | 0 | 0 | 0 | 0 | 0 | 0 |
| | Red No.102 | 0 | 0 | 0 | 0 | 0 | 0.5 | 0 | 0 |
| Solvent ratio in coating solution (mass%) for outer layer (second layer) | Water | 10 | 30 | 50 | 10 | 30 | 10 | 10 | 10 |
| | EtOH | 90 | 70 | 50 | 90 | 70 | 90 | 90 | 90 |
| | IPA | 0 | 0 | 0 | 0 | 0 | 0 | 0 | 0 |
| | Acetone | 0 | 0 | 0 | 0 | 0 | 0 | 0 | 0 |
| Solid concentration in coating solution (mass%) for outer layer (second layer) | | 14.5 | 12.1 | 13.0 | 19.0 | 12.9 | 14.8 | 17.7 | 17.8 |
| Shear viscosity of coating solution (cP) for outer layer (second layer) | | 888 | 670 | 1217 | 772 | 550 | 638 | 451 | 764 |
| Whether band seal is applied or not | | Applied | Applied | Applied | Applied | Applied | Applied | Applied | Applied |
| Water dissolution test | Dissolution rate after 120 minutes (%) | - | 0.6 | 0.4 | 0.3 | 0.3 | 0.1 | 0.1 | 0.0 |
| pH5.0 dissolution test | Dissolution rate after 120 minutes (%) | 0.2 | 0.3 | - | 0.1 | - | - | - | 0.7 |
| pH6.0 dissolution test | Dissolution rate after 120 minutes (%) | - | - | - | - | 0.4 | 0.9 | - | - |

Fig. 10

| | | Comparative example 20 | Comparative example 21 |
|---|---|---|---|
| | | - | - |
| Inner layer (first layer) | Base | HPMC | HPMC |
| | Gelling Agent | CA | CA |
| | Thickness (μm) | 80 | 90 |
| Outer layer (second layer) | Enteric polymer | AS-LG | AS-HG |
| | Thickness (μm) | 30 | 40 |
| | Structure | A | A |
| Composition ratio of outer layer (second layer) shell (mass%) | Enteric polymer | 100 | 100 |
| Solvent ratio in coating solution (mass%) for outer layer (second layer) | Water | 98.6 | 99.2 |
| | 28 vol% ammonia solution | 1.4 | 0.8 |
| Solid concentration in coating solution (mass%) for outer layer (second layer) | | 11.9 | 12.2 |
| Shear viscosity of coating solution (cP) for outer layer (second layer) | | 1028 | 1414 |
| Whether band seal is applied or not | | Applied | Applied |
| First fluid (pH1.2) dissolution test | Dissolution rate after 120 minutes (%) | 1.3 | 0.7 |
| Second fluid (pH6.8) dissolution test | Dissolution rate after 30 minutes (%) | - | - |
| Water dissolution test | Dissolution rate after 120 minutes (%) | 19.0 | 5.1 |
| pH5.0 dissolution test | Dissolution rate after 120 minutes (%) | 6.7 | - |
| pH6.0 dissolution test | Dissolution rate after 120 minutes (%) | - | 17.5 |

Fig. 11

| | Base | Thickness of first layer (μm) | Thickness of second layer (μm) | Quantity of NH4+ (g/shell100g) | Degree of neutralization (mol%) |
|---|---|---|---|---|---|
| Comparative example 20 | AS-LG | 80 | 30 | 0.1100 | 16.09 |
| Comparative example 21 | AS-HG | 90 | 40 | 0.0630 | 16.67 |
| Example 1 | AS-LG | 90 | 30 | 0.001 or less | 0.16 |
| Example 30 | AS-HG | 70 | 50 | 0.001 or less | 0.20 |
| Example 12 | HP-55 | 80 | 20 | 0.0011 | 0.15 |
| Example 32 | CMEC | 70 | 30 | 0.001 or less | 0.09 |
| Example 34 | AS-HG | 80 | 30 | 0.001 or less | 0.29 |
| Example 20 | AS-LG | 80 | 40 | 0.001 or less | 0.21 |
| Example 22 | AS-LG | 120 | 30 | 0.001 or less | 0.24 |

## INTERNATIONAL SEARCH REPORT

| International application No. |
|---|
| **PCT/JP2023/026717** |

**A. CLASSIFICATION OF SUBJECT MATTER**

***A61K 9/48***(2006.01)i; ***A61K 47/02***(2006.01)i; ***A61K 47/12***(2006.01)i; ***A61K 47/14***(2017.01)i; ***A61K 47/20***(2006.01)i; ***A61K 47/32***(2006.01)i; ***A61K 47/36***(2006.01)i; ***A61K 47/38***(2006.01)i; ***A61K 47/42***(2017.01)i; ***A61K 31/167***(2006.01)i; ***A61P 29/00***(2006.01)i

FI: A61K9/48; A61K47/38; A61K47/14; A61K47/02; A61K47/20; A61K47/42; A61K47/36; A61K47/32; A61K31/167; A61K47/12; A61P29/00

According to International Patent Classification (IPC) or to both national classification and IPC

**B. FIELDS SEARCHED**

Minimum documentation searched (classification system followed by classification symbols)

A61K9/48; A61K47/02; A61K47/12; A61K47/14; A61K47/20; A61K47/32; A61K47/36; A61K47/38; A61K47/42; A61K31/167; A61P29/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 49-30524 A (SUGIMOTO, Isao) 19 March 1974 (1974-03-19)<br>claims, p. 2, upper left column, line 14 to p. 3, upper left column, line 7, examples | 1-20 |
| X | JP 2021-503463 A (EVONIK OPERATIONS GMBH) 12 February 2021 (2021-02-12)<br>claims, paragraphs [0043]-[0067], [0096]-[0107], [0125]-[0131], examples | 1-20 |
| X | JP 61-221117 A (FUJISAWA PHARMACEUTICAL CO) 01 October 1986 (1986-10-01)<br>claims, p. 2, upper right column, lines 9-19, examples | 1-20 |
| X | JP 63-117761 A (TSUJI, Shinjiro) 21 May 1988 (1988-05-21)<br>claims, p. 2, left column, lines 1-12, examples | 1-20 |
| X | JP 63-22014 A (SHIONOGI & CO LTD) 29 January 1988 (1988-01-29)<br>claims, reference examples 3-5 | 1-20 |

☑ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **01 September 2023** | **19 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

# EP 4 559 458 A1

## INTERNATIONAL SEARCH REPORT

| | International application No. |
|---|---|
| | **PCT/JP2023/026717** |

### C. DOCUMENTS CONSIDERED TO BE RELEVANT

| Category* | Citation of document, with indication, where appropriate, of the relevant passages | Relevant to claim No. |
|---|---|---|
| X | JP 2003-306428 A (ZENSEI YAKUHIN KOGYO KK) 28 October 2003 (2003-10-28) claims, paragraph [0008], example 2, treatment O | 1-20 |
| X | JP 2007-230948 A (UNIV MEIJO) 13 September 2007 (2007-09-13) claims, paragraphs [0007]-[0009], [0038]-[0043], [0054], example 5 | 1-20 |
| X | HUYGHEBAERT, N. et al. Alternative method for enteric coating of HPMC capsules resulting in ready-to-use enteric-coated capsules. European Journal of Pharmaceutical Sciences. 2004, vol. 21, pp. 617-623 abstract, sections 2.1.2-2.1.3.2, 3.2, fig. 2-4 | 1-20 |

Form PCT/ISA/210 (second sheet) (January 2015)

39

**INTERNATIONAL SEARCH REPORT**
Information on patent family members

International application No.

**PCT/JP2023/026717**

| Patent document cited in search report | | | Publication date (day/month/year) | Patent family member(s) | Publication date (day/month/year) |
|---|---|---|---|---|---|
| JP | 49-30524 | A | 19 March 1974 | (Family: none) | |
| JP | 2021-503463 | A | 12 February 2021 | US 2021/0361585 A1 claims, paragraphs [0063]-[0103], [0135]-[0146], [0168]-[0177], examples WO 2019/096833 A1 EP 3709979 A1 | |
| JP | 61-221117 | A | 01 October 1986 | (Family: none) | |
| JP | 63-117761 | A | 21 May 1988 | (Family: none) | |
| JP | 63-22014 | A | 29 January 1988 | (Family: none) | |
| JP | 2003-306428 | A | 28 October 2003 | (Family: none) | |
| JP | 2007-230948 | A | 13 September 2007 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6309666 B
- US 7094425 B
- WO 2020229178 A
- WO 2020229192 A
- JP 2003325642 A
- US 3927195 A
- JP 5686802 B
- JP 2006016372 A
- WO 2019245031 A
- JP 2015518005 W
- JP 2010202550 A
- JP 2009196961 A

**Non-patent literature cited in the description**

- Japanese Pharmacopoeia